(19) Europäisches Patentamt
European Patent Office
Office européen des brevets

(11) Veröffentlichungsnummer : **0 139 612**
**B1**

(12) # EUROPÄISCHE PATENTSCHRIFT

(45) Veröffentlichungstag der Patentschrift : **10.02.88**

(21) Anmeldenummer : **84810410.5**

(22) Anmeldetag : **20.08.84**

(51) Int. Cl.⁴ : **C 07 D239/46**, C 07 D417/12, C 07 D403/12, C 07 D401/12, C 07 F 9/65, A 01 N 47/36, A 01 N 57/16// C07D239/42

(54) **Herbizide Mittel.**

(30) Priorität : **26.08.83 CH 4669/83**

(43) Veröffentlichungstag der Anmeldung : **02.05.85 Patentblatt 85/18**

(45) Bekanntmachung des Hinweises auf die Patenterteilung : **10.02.88 Patentblatt 88/06**

(84) Benannte Vertragsstaaten : **BE CH DE FR GB IT LI**

(56) Entgegenhaltungen :
**EP-A- 0 084 224**
**US-A- 4 169 719**

(73) Patentinhaber : **CIBA-GEIGY AG**
**Klybeckstrasse 141**
**CH-4002 Basel (CH)**

(72) Erfinder : **Töpfl, Werner, Dr.**
**Dorneckstrasse 68**
**CH-4143 Dornach (CH)**

**EP 0 139 612 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue, herbizid wirksame und pflanzenwuchsregulierende N-Arylsulfonyl-N'-(4-mercaptomethyl-pyrimidinyl- und -triazinyl)-harnstoff-Derivate, Verfahren zu ihrer Herstellung, sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums. Darüberhinaus betrifft die Erfindung auch als Zwischenprodukte hergestellte neue 2-Amino-4-mercaptomethyl-pyrimidin- und -triazin-Derivate.

Die erfindungsgemässen N-Arylsulfonyl-N'-(4-mercaptomethyl-pyrimidinyl- und -triazinyl)-harnstoffe entsprechen der Formel I

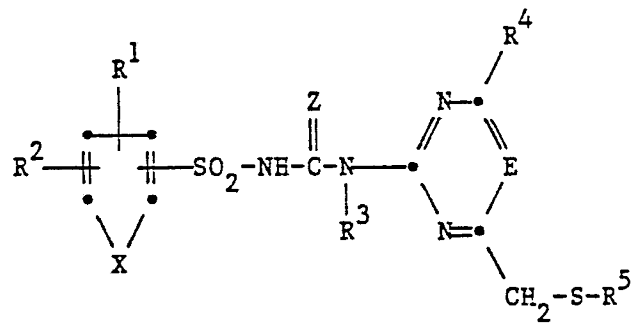

(I)

worin

E Stickstoff oder —CH=,

X Sauerstoff, Schwefel, $—NR^3—$, $—N=CR^3—$, —CH=CH— oder

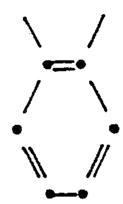

.

Z Sauerstoff oder Schwefel,

$R^1$ Wasserstoff, Halogen, Nitro, Aethinyl, $—NR^{16}R^{17}$, $—CR^6$-di-$C_1$-$C_4$-Alkoxy,

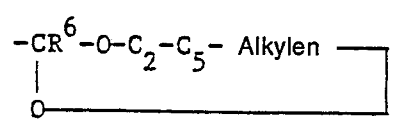

$—CW—R^6$, $—SO_2—NR^7R^8$, $—CO—R^9$, $—Y_m—R^{10}$, $—SO_2—R^{11}$ oder $—O—SO_2R^{12}$, wobei m für Null oder eins steht,

$R^2$ Wasserstoff, Halogen, $C_1$-$C_4$-Alkyl, $C_1$-$C_4$-Alkoxy, $C_1$-$C_4$-Halogenalkyl oder Nitro,

$R^3$ Wasserstoff, $C_1$-$C_4$-Alkyl, $C_3$-$C_4$-Alkenyl oder $C_1$-$C_4$-Alkoxy,

$R_4$ Wasserstoff, Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylthio, $C_2$-$C_4$-Alkoxyalkyl, $C_2$-$C_4$-Alkoxyalkoxy, Cyclopropyl, $NH_2$, $C_1$-$C_4$-Alkylamino, Di-$C_1$-$C_4$-alkylamino oder einen über das Stickstoffatom gebundenen gesättigten 5- bis 7-gliedrigen Stickstoffheterocyclus, der noch ein weiteres Heteroatom enthalten kann, Cyan, $—CZ—R^{13}$,

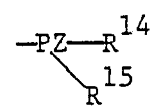

oder einen unsubstituierten oder einen durch einen Rest aus der Gruppe Halogen, $C_1$-$C_4$-Alkyl, $C_1$-$C_4$-Alkoxy oder $C_1$-$C_4$-Halogenalkyl substituierten ungesättigten Heterocyclus ausgewählt aus Reihe Imidazol, Triazol, Pyridin, Pyrimidin, Thiazol, Oxazol, Thiadiazol, Oxadiazol, Pyridazin, Thiophen oder Furan oder deren teilhydrierten Abkömmlingen,

$R^6$ Wasserstoff, $C_1$-$C_4$-Alkyl, $C_1$-$C_4$-Halogenalkyl, $C_3$-$C_6$-Cycloalkyl, $C_4$-$C_7$-Cycloalkylalkyl oder $C_2$-$C_4$-Alkoxyalkyl,

$R^7$ und $R^{16}$ unabhängig voneinander Wasserstoff, $C_1$-$C_4$-Alkyl, $C_1$-$C_4$-Cyanoalkyl oder $C_1$-$C_4$-Alkoxy,

$R^8$ und $R^{17}$ unabhängig voneinander Wasserstoff, $C_1$-$C_4$-Alkyl, $C_3$-$C_4$-Alkenyl oder $C_1$-$C_4$-Alkoxy, oder

$R^7$ und $R^8$ sowie $R^{16}$ und $R^{17}$ unabhängig voneinander zusammen mit dem sie bindenden Stickstoffatom einen 5- bis 7-gliedrigen gesättigten Stickstoffheterocyclus, der noch ein weiteres Heteroatom enthalten kann,

$R^9$ $C_1$-$C_4$-Alkoxy, $C_3$-$C_6$-Alkenyloxy, $C_3$-$C_6$-Alkinyloxy, $C_2$-$C_6$-Halogenalkoxy, $C_1$-$C_4$-Cyanalkoxy, $C_1$-$C_4$-Alkylthio. $C_3$-$C_4$-Alkenylthio, $C_3$-$C_4$-Alkinylthio, $C_5$-$C_6$-Cycloalkoxy, $C_4$-$C_7$-Cycloalkylalkoxy, —$NR^7R^8$ oder $C_2$-$C_6$-Alkoxyalkoxy,

$R^{10}$ $C_3$-$C_4$-Alkinyl, $C_2$-$C_4$-Alkenyl, $C_1$-$C_4$-Alkyl, ein- oder mehrfach durch Halogen, Cyan, $C_1$-$C_4$-Alkoxy oder —$SO_n$-$C_1$-$C_4$-Alkyl substituiertes $C_2$-$C_4$-Alkenyl oder ein- oder mehrfach durch Halogen, Cyan, Nitro, $C_1$-$C_4$-Alkoxy, $C_1$-$C_4$-Halogenalkoxy,- $C_1$-$C_4$-Halogenalkylthio, —$SO_n$-$C_1$-$C_4$-Alkyl, —T—CX—$R^{18}$,

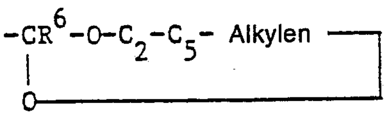

—CO—$R^6$, —CO—$R^9$, oder —$SO_2$—$NR^7R^8$ substituiertes $C_1$-$C_4$-Alkyl, wobei n für Null, 1 oder 2 steht,

$R^{11}$ $C_2$-$C_4$-Halogenalkoxy,

$R^{12}$ $C_1$-$C_4$-Alkyl, $C_1$-$C_4$-Halogenalkyl oder —$NR^{16}R^{17}$,

$R^{13}$ $C_1$-$C_4$-Alkyl, $C_1$-$C_4$-Alkoxy, $C_1$-$C_4$-Alkylthio, Phenyl, Di-$C_1$-$C_4$-alkylamino oder über das Stickstoffatom gebundenen gesättigten 5- bis 7-gliedrigen Stickstoffheterocyclus, der noch ein weiteres Heteroatom enthalten kann,

$R^{14}$ und $R^{15}$ unabhängig voneinander $C_1$-$C_4$-Alkyl, $C_1$-$C_4$-Alkoxy, $C_3$-$C_4$-Alkoxyalkoxy, $C_3$-$C_4$-Alkenyloxy, $C_1$-$C_6$-Alkylthio, $C_1$-$C_4$-Alkylamino oder Di-$C_1$-$C_4$-alkylamino,

$R^{18}$ $C_1$-$C_4$-Alkyl, $C_1$-$C_4$-Halogenalkyl, $C_1$-$C_4$-Alkoxy, $C_1$-$C_4$-Alkylthio oder —$NR^{16}R^{17}$,

T Sauerstoff oder Schwefel,

W Sauerstoff oder =N—O—$R^3$ und

Y Sauerstoff, Schwefel, —SO— oder —$SO_2$—

bedeuten, sowie den Salzen dieser Verbindungen.

Harnstoffverbindungen, Triazinverbindungen und Pyrimidinverbindungen mit herbizider Wirkung sind allgemein bekannt. Kürzlich wurden Sulfonylharnstoffverbindungen mit herbizider und pflanzenwuchsregulierender Wirkung, beispielsweise in den europäischen Patentanmeldungen 44807 und 44808 beschrieben.

In den Definitionen ist unter Alkyl geradkettiges oder verzweigtes Alkyl zu verstehen, z. B. : Methyl, Aethyl, n-Propyl, i-Propyl oder die vier isomeren Butyl.

Unter Alkoxy ist zu verstehen : Methoxy, Aethoxy, n-Propyloxy, i-Propyloxy, die vier isomeren Butyloxyreste, n-Amyloxy, i-Amyloxy, 2-Amyloxy oder 3-Amyloxy, insbesondere aber Methoxy, Aethoxy oder i-Propyloxy.

Beispiele für Alkylthio sind Methylthio, Aethylthio, n-Propylthio, i-Propylthio, n-Butylthio oder n-Pentylthio, insbesondere aber Methylthio oder Aethylthio.

Beispiele für Alkylsulfinyl sind Methylsulfinyl, Aethylsulfinyl, n-Propylsulfinyl und n-Butylsulfinyl, insbesondere aber Methylsulfinyl und Aethylsulfinyl.

Beispiele für Alkylsulfonyl sind Methylsulfonyl, Aethylsulfonyl oder n-Propylsulfonyl, insbesondere aber Methylsulfonyl und Aethylsulfonyl.

Unter Halogen in den Definitionen selbst sowie Halogen als Teil in Halogenalkoxy, Halogenalkyl oder Halogenalkylthio sind Fluor, Chlor und Brom, vorzugsweise jedoch Fluor oder Chlor, zu verstehen.

Die an die Sulfonylbrücke gebundenen Arylteile der Wirkstoffe der Formel I sind durch folgende zugrundeliegende aromatische Ringsysteme charakterisiert : Thiophen, Furan, Pyridin, Pyrrol, Phenyl oder Naphthalin. Bevorzugt ist der Phenylring.

Beispiele für die unter $R^4$ und $R^{13}$ definierten Stickstoffheterocyclen sind über das Stickstoffatom gebundene Ringe der folgenden Grundtypen : Pyrrolidin, Piperidin, Morpholin, Thiomorpholin oder Piperazin oder Hexamethylenimin.

Bevorzugte ungesättigte Heterocyclensubstituenten innerhalb der Definition von $R^5$ sind 4,5-Dihydrothiazol-2-yl, 2H-1,2,4-Triazol-3-yl, 1-Methyl-imidazol-2-yl, 2-Pyridinyl und 2-Pyrimidinyl.

Beispiele für Cycloalkyl sind Cyclopropyl, Cyclobutyl, Cyclopentyl und Cyclohexyl, vorzugsweise aber Cyclopentyl und Cyclohexyl. Bevorzugte Cycloalkylalkylreste sind Cyclopropylmethyl, Cyclopentylmethyl und Cyclohexylmethyl. Alkoxyalkylreste werden repräsentiert durch Methoxymethyl, Aethoxymethyl, Methoxyäthyl und Aethoxyäthyl, insbesondere aber Methoxyäthyl. Alkoxyalkoxyreste sind im Rahmen der vorliegenden Erfindung Methoxymethoxy, Aethoxymethoxy, Methoxyäthoxy und Aethoxy-

äthoxy. Bevorzugte Cyanoalkylreste sind Cyanomethyl und Cyanoäthyl. Halogenalkyl als Substituent selbst oder als Teil eines anderen Substituenten wie Halogenalkoxy oder Halogenalkylthio steht in der Regel für Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl. Chlormethyl, Difluormethyl und Trifluormethyl.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Aethylamin, Propylamin, i-Propylamin, die vier isomeren Butylamine. Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Unter den Verbindungen der Formel I sind diejenigen bevorzugt, in denen entweder

a) X für die Aethenylenbrücke —CH=CH— steht, oder

b) Z für Sauerstoff steht, oder

c) $R^1$ für $C_1$-$C_4$-Alkoxycarbonyl, Nitro, Halogen, $C_1$-$C_4$-Halogenalkyl, $C_1$-$C_4$-Halogenalkoxy oder Di-$C_1$-$C_4$-alkylsulfamoyl steht, oder

d) $R^2$ Wasserstoff bedeutet, oder

e) $R^3$ Wasserstoff bedeutet, oder

f) $R^4$ für Halogen, $C_1$-$C_4$-Alkoxy oder $C_1$-$C_4$-Alkyl steht, oder

g) E für die Methinbrücke —CH= steht, oder

h) $R^5$ für $C_1$-$C_4$-Alkylcarbonyl, $C_1$-$C_4$-Alkoxycarbonyl, $C_1$-$C_4$-Alkoxythiocarbonyl, Di-$C_1$-$C_4$-alkylcarbamoyl, Di-$C_1$-$C_4$-alkylthiocarbamoyl, Di-$C_1$-$C_4$-alkoxyphosphonyl, Di-$C_1$-$C_4$-alkoxythiophosphonyl, N-Pyrrolidinocarbonyl, N-Pyrrolidinothiocarbonyl, N-Morpholinothiocarbonyl, 2H-1,2,4-Triazol-3-yl, 4,5-Dihydrothiazol-2-yl, 1-$C_1$-$C_4$-Alkyl-imidazol-2-yl, 2-Pyridinyl oder 2-Pyrimidinyl steht.

Eine weiter bevorzugte Untergruppe bilden solche Verbindungen der Formel I, in denen X die Aethenylenbrücke, Z Sauerstoff, $R^1$ $C_1$-$C_4$-Alkoxycarbonyl, Nitro, Halogen, $C_1$-$C_4$-Halogenalkyl, $C_1$-$C_4$-Halogenalkoxy oder Di-$C_1$-$C_4$-Alkylsulfamoyl und $R^2$ und $R^3$ Wasserstoff bedeuten.

Ebenso bevorzugt sind die Verbindungen der Formel I, in denen $R^4$ Halogen, $C_1$-$C_4$-Alkoxy oder $C_1$-$C_4$-Alkyl, E die Methinbrücke und $R^5$ $C_1$-$C_4$-Alkylcarbonyl, $C_1$-$C_4$-Alkoxycarbonyl, $C_1$-$C_4$-Alkoxythiocarbonyl, Di-$C_1$-$C_4$-alkylcarbamoyl, Di-$C_1$-$C_4$-alkylthiocarbamoyl, Di-$C_1$-$C_4$-alkoxyphosphonyl, Di-$C_1$-$C_4$-alkoxythiophosphonyl, N-Pyrrolidinocarbonyl, N-Pyrrolidinothiocarbonyl, N-Morpholinothiocarbonyl, 2H-1,2,4-Triazol-3-yl, 4,5-Dihydrothiazol-2-yl, 1-$C_1$-$C_4$-Alkyl-imidazol-2-yl, 2-Pyridinyl oder 2-Pyrimidinyl bedeuten.

Eine besonders bevorzugte Untergruppe von Verbindungen der Formel I bilden diejenigen, in denen X die Aethenylenbrücke, Z Sauerstoff, $R^1$ $C_1$-$C_4$-Alkoxycarbonyl, Nitro, Halogen, $C_1$-$C_4$-Halogenalkyl, $C_1$-$C_4$-Halogenalkoxy oder Di-$C_1$-$C_4$-Alkylsulfamoyl und $R^2$ und $R^3$ Wasserstoff. Halogen, $C_1$-$C_4$-Alkoxy oder $C_1$-$C_4$-Alkyl, E die Methinbrücke und $R^5$ $C_1$-$C_4$-Alkylcarbonyl, $C_1$-$C_4$-Alkoxycarbonyl, $C_1$-$C_4$-Alkoxythiocarbonyl, Di-$C_1$-$C_4$-alkylcarbamoyl, Di-$C_1$-$C_4$-alkylthiocarbamoyl, Di-$C_1$-$C_4$-alkoxyphosphonyl, Di-$C_1$-$C_4$-alkoxythiophosphonyl, N-Pyrrolidinocarbonyl, N-Pyrrolidinothiocarbonyl, N-Morpholinothiocarbonyl, 2H-1,2,4-Triazol-3-yl, 4,5-Dihydrothiazol-2-yl, 1-$C_1$-$C_4$-Alkyl-imidazol-2-yl, 2-Pyridinyl oder 2-Pyrimidinyl bedeuten.

Als bevorzugte Einzelverbindungen sind zu nennen :

N-(2-Methoxycarbonylphenyl-sulfonyl)-N'-(4-methoxy-6-acetylthiomethyl-pyrimidin-2-yl)-harnstoff,

N-(2-Methoxycarbonylphenyl-sulfonyl)-N'-(4-methoxy-6-methoxythiocarbonylthiomethyl-pyrimidin-2-yl)-harnstoff,

N-(2-Nitrophenyl-sulfonyl)-N'-[4-methoxy-6-(4,5-dihydrothiazol-2-yl-thiomethyl)-pyrimidin-2-yl]-harnstoff,

N-(2-Methoxycarbonylphenyl-sulfonyl)-N'-(4-methoxy-6-dimethoxythiophosphonylthiomethyl-pyrimidin-2-yl)-harnstoff,

N-(2-Nitrophenyl-sulphonyl)-N'-(4-methoxy-6-diäthoxyphosphonylthiomethyl-pyrimidin-2-yl)-harnstoff und

N-(2-Methoxycarbonylphenyl-sulfonyl)-N'-(4-methoxy-6-di-n-butyloxythiophosphonylthiomethyl-pyrimidin-2-yl)-harnstoff.

Die Herstellung der Verbindungen der Formel I erfolgt im allgemeinen nach den folgenden Methoden.

Nach einem ersten Verfahren werden die Verbindungen der Formel I erhalten, indem man ein

Arylsulfonylisocyanat oder -isothiocyanat der Formel II

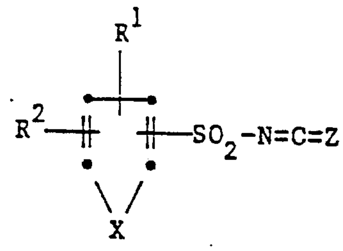

(II)

worin $R^1$, $R^2$, X und Z die unter Formel I gegebene Bedeutung haben, mit einem Aminopyrimidin oder -triazin der Formel III

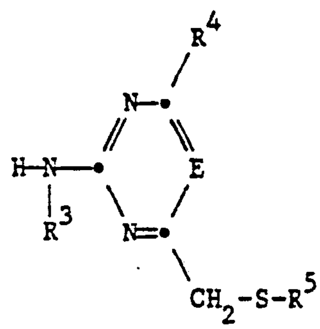

(III)

worin $R^3$, $R^4$, $R^5$ und E die unter Formel I gegebene Bedeutung haben, umsetzt.

Nach einem weiteren Verfahren erhält man die Verbindungen der Formel I, indem man ein N-Arylsulfonylcarbamat der Formel IV

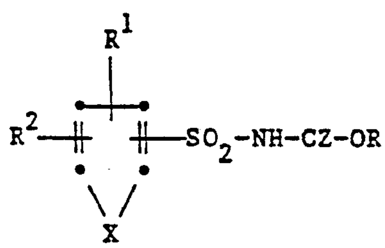

(IV)

worin $R^1$, $R^2$, X und Z die unter Formel I gegebene Bedeutung haben, und R für Phenyl, Alkyl oder substituiertes Phenyl steht, mit einem Aminopyrimidin oder -triazin der Formel III umsetzt.

Schliesslich kann man die Verbindungen der Formel I auch herstellen, indem man eine Verbindung der Formel V

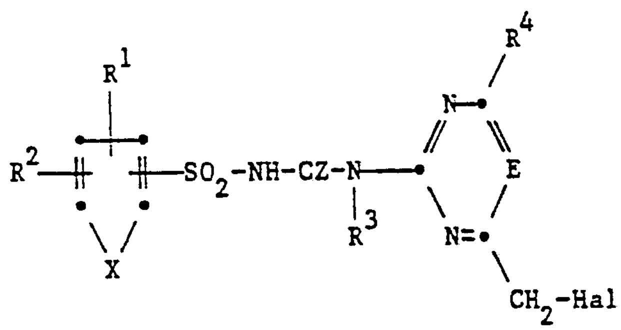

(V)

worin $R^1$, $R^2$, $R^3$, $R^4$, X und Z die unter Formel I gegebene Bedeutung haben, und Hal für Chlor oder Brom steht, mit einem Schwefelsalz der Formel VI

$$M^{\oplus}\ {}^{\ominus}S - R^5 \qquad \text{(VI)},$$

worin $R^5$ die unter Formel I gegebene Bedeutung hat und $M^{\oplus}$ für ein Alkali-, Erdalkali- oder Ammoniumkation steht, umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Methylenchlorid, Chloroform, Tetrachlorkohlenstoff, oder Chlorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidin. Die Reaktionstemperaturen liegen vorzugsweise zwischen —20° und + 120 °C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können mit Vorteil gewünschtenfalls durch Zugabe einiger Tropfen Base als Reaktionskatalysator verkürzt werden. Als Basen sind dazu insbesondere tertiäre Amine wie Trimethylamin, Triäthylamin, Chinuclidin, 1,4-Diazabicyclo-(2,2,2)-octan, 1,5-Diazabicyclo(4,3,0) non-5-en oder 1,5-Diazabicyclo(5,4,0) undec-7-en geeignet. Als Basen können aber auch anorganische Basen wie Hydride, wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat, verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen, gereinigt werden.

Die Zwischenprodukte der Formeln II, IV, V und VI sind bekannt oder können analog zu bekannten Methoden hergestellt werden.

Die Aminopyrimidine und -triazine der Formel III sind neu und wurden speziell als Zwischenprodukte zur Herstellung der erfindungsgemässen Wirkstoffe entwickelt und hergestellt. Sie bilden daher einen weiteren Gegenstand der vorliegenden Erfindung.

Die neuen Aminopyrimidine und -triazine der Formel III werden hergestellt, indem man eine Verbindung der Formel VII

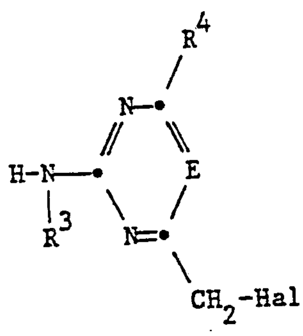

(VII)

worin $R^3$, $R^4$ und E die unter Formel I gegebene Bedeutung haben und Hal für Chlor oder Brom steht, mit einem Schwefelsalz der Formel VI umsetzt.

Diese Reaktion wird unter den gleichen Reaktionsbedingungen durchgeführt wie die Umsetzung der Verbindungen V und VI.

Die Wirkstoffe der Formel I sind stabile Verbindungen. Ihre Handhabung bedarf keiner vorsorglicher Massnahmen.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d. h. sie werden von den Pflanzen aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei — im Vergleich zu anderen Herbiziden und Wuchsregulatoren — sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z. B. die in der Landwirtschaft in tropischen Gegenden häufig als « cover crops » (Bodenbedecker) angepflanzten Leguminosen durch die Verbindungen der Formel I in ihrem Wachstum

selektiv gehemmt werden, so dass zwar die Bodenerosion zwischen den Kulturpflanzen verhindert wird, die « cover crops » jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfonate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. die Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und

6 bis 18 Kohlenstoffatome im Alkylrest der Alkylphenole enthalten können.

Weitere geeignete nichtionische Tenside sind die wasserlöslichen, 20 bis 250 Aethylenglykolăthergruppen und 10 bis 100 Propylenäthergruppen enthaltenden Polyäthyleoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ridgewood, New Jersey, 1981 ;

H. Stache, « Tensid-Taschenbuch », 2. Aufl., C. Hanser Verlag, München, Wien, 1981 ;

M. and J. Ash. « Encyclopedia of Surfactants », Vol. I-III, Chemical Publishing Co., New York, 1980-1981.

Die pestiziden Zubereitungen enthalten in der Regel 0,1 bis 95 %, insbesondere 0,1 bis 80 % Wirkstoff der Formel I, 1 bis 99 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen : (% = Gewichtsprozent)

| | |
|---|---|
| Emulgierbare Konzentrate : | |
| Aktiver Wirkstoff | 1 bis 20 %, bevorzugt 5 bis 10 % |
| oberflächenaktives Mittel | 5 bis 30 %, vorzugsweise 10 bis 20 % |
| flüssiges Trägermittel | 50 bis 94 %, vorzugsweise 70 bis 85 % |
| Stäube : | |
| Aktiver Wirkstoff | 0,1 bis 10 %, vorzugsweise 0,2 bis 1 % |
| festes Trägermittel | 99,9 bis 90 %, vorzugsweise 99,9 bis 99 % |
| Suspensions-Konzentrate : | |
| Aktiver Wirkstoff | 5 bis 75 %, vorzugsweise 10 bis 50 % |
| Wasser | 94 bis 25 %, vorzugsweise 90 bis 30 % |
| oberflächenaktives Mittel | 1 bis 40 %, vorzugsweise 2 bis 30 % |
| Benetzbare Pulver : | |
| Aktiver Wirkstoff | 0,5 bis 90 %, vorzugsweise 1 bis 80 % |
| oberflächenaktives Mittel | 0,5 bis 20 %, vorzugsweise 1 bis 15 % |
| festes Trägermaterial | 5 bis 95 %, vorzugsweise 15 bis 90 %. |
| Granulate : | |
| Aktiver Wirkstoff | 0,5 bis 30 %, vorzugsweise 3 bis 15 %. |
| festes Trägermittel | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### Beispiel H1

N-(2-Methoxycarbonylphenyl-sulfonyl)-N'-(4-methoxy-6-äthoxythiocarbonylthiomethyl-pyrimidin-2-yl)-harnstoff (Verbindung Nr. 2.4)

7,0 g 2-Amino-4-methoxy-6-äthoxythiocarbonylthiomethyl-pyrimidin und 6,5 g 2-Methoxycarbonylphenylsulfonylisocyanat werden in 20 ml abs. Acetonitril suspendiert und für 2 Stunden bei 20 bis 25 °C gerührt. Während dieser Zeit entsteht zunächst eine klare Lösung und später kristallisiert das Endprodukt aus. Zur Isolierung des Endprodukts wird das Reaktionsgemisch auf 0 °C abgekühlt und filtriert. Man erhält so 9,0 g (67 % d. Th.) N-(2-Methoxycarbonylphenyl-sulfonyl)-N'-(4-methoxy-6-äthoxythiocarbonylthiomethyl-pyrimidin-2-yl)-harnstoff, Smp. 160-162 °C (Zers.)

Beispiel H2

N-(2-Dimethylsulfamoylphenyl-sulfonyl)-N'-(4-methoxy-6-dimethylthiocarbamoylthiomethyl-pyrimidin-2-yl)-harnstoff (Verbindung Nr. 2.7)

Eine Mischung von 9,3 g N-(2-Dimethylsulfamoylphenyl-sulfonyl)-N'-(4-chlormethyl-6-methoxy-pyrimidin-2-yl)-harnstoff und 3,7 g Dimethyldithiocarbamidsäure Natriumsalz Dihydrat wird in 100 ml Acetonitril bei 20-25 °C für 3 Stunden gerührt. Zur Aufarbeitung wird das Reaktionsgemisch mit 1 Liter Wasser verdünnt und das ausgefallene Produkt abgetrennt. Man erhält so 10,0 g (91 % d. Th.) N-(2-Dimethylsulfamoylphenyl-sulfonyl)-N'-(4-methoxy-6-dimethylthiocarbamoylthiomethyl-pyrimidin-2-yl)-harnstoff, Smp. 181-183 °C (Zers.).

Beispiel H3

N-(2-Nitrophenyl-sulfonyl)-N'-(4-methoxy-6-dimethylthiocarbamoylthiomethyl-pyrimidin-2-yl)-harnstoff (Verbindung Nr. 2.10)

7,0 g 2-Amino-4-methoxy-6-dimethylcarbamoylthiomethyl-pyrimidin und 6,2 g 2-Nitrophenylsulfonylisocyanat werden in 80 ml abs. Acetonitril gelöst und für 50 Stunden bei Raumtemperatur gerührt. Das Produkt scheidet sich während dieses Zeitraumes teilweise kristallin ab. Nach dem Abkühlen des Reaktionsgemisches auf 0 °C wird der Niederschlag abgetrennt. Man erhält so 10,0 g (76 % d. Th.) N-(2-Nitrophenyl-sulfonyl)-N'-(4-methoxy-6-dimethylthiocarbamoylthiomethyl-pyrimidin-2-yl)-harnstoff, Smp. 202-204 °C (Zers.).

Beispiel H4

N-(2-Methoxycarbonylphenyl-sulfonyl)-N'-[4-methoxy-6-(4,5-dihydrothiazol-2-yl-thiomethyl)-pyrimidin-2-yl]-harnstoff (Verbindung Nr. 2.15)

7,0 g 2-Amino-4-methoxy-6-(4,5-dihydrothiazol-2-yl)-pyrimidin werden in 50 ml abs. Acetonitril suspendiert und mit 6,6 g 2-Methoxycarbonylphenylsulfonylisocyanat versetzt. Dabei entsteht unter Erwärmung eine klare Lösung. Anschliessend scheidet sich das Produkt kristallin ab. Nachdem das Reaktionsgemisch für 3 Stunden bei 20 bis 25 °C gerührt worden ist, wird es auf 0 °C abgekühlt und der Feststoff abgetrennt. Man erhält so N-(2-Methoxycarbonylphenyl-sulfonyl)-N'-[4-methoxy-6-(4,5-dihydrothiazol-2-yl)-thiomethyl-pyrimidin-2-yl]-harnstoff. Smp. 135-145 °C (Zers.).

Beispiel H5

N-(2-Nitrophenyl-sulfonyl)-N'-(4-methoxy-6-di-n-propyloxythiophosphonylthiomethyl-pyrimidin-2-yl)-harnstoff (Verbindung Nr. 2.23)

Eine Mischung von 11,6 g 2-Amino-4-methoxy-di-n-propyloxythiophosphonylthiomethyl-pyrimidin und 7,5 g 2-Nitrophenylsulfonylisocyanat wird in 30 ml abs. Acetonitril gerührt. Nach kurzer Zeit scheidet sich aus diesem Gemisch das Produkt ab. Das Reaktionsgemisch wird für weitere 20 Stunden gerührt und auf 0 °C abgekühlt. Durch Abtrennen des Niederschlages erhält man 6,5 g (34 % d. Th.) N-(2-Nitrophenylsulfonyl)-N'-(4-methoxy-6-di-n-propyloxythiophosphonylthiomethyl-pyrimidin-2-yl)-harnstoff, Smp. 157-159 °C.

Beispiel H6

N-(2-Methoxycarbonylphenyl-sulfonyl)-N'-(4-methoxy-6-di-i-propyloxythiophosphonylthiomethyl-pyrimidin-2-yl)-harnstoff (Verbindung Nr. 2.24)

Ein Gemisch von 11,0 g 2-Amino-4-methoxy-6-di-i-propyloxythiophosphonylthiomethyl-pyrimidin und 7,0 g 2-Methoxycarbonylphenylsulfonylisocyanat wird in 50 ml abs. Acetonitril für 20 Stunden bei 20 bis 25 °C gerührt. Anschliessend wird das Lösungsmittel im Vakuum abgedampft und der ölige Rückstand aus Diäthyläther kristallisiert. Man erhält so 11,0 g (60 % d. Th.) N-(2-Methoxycarbonylphenylsulfonyl)-N'-(4-methoxy-6-di-i-propyloxythiophosphonylthiomethyl-pyrimidin-2-yl)-harnstoff, Smp. 119-123 °C.

Beispiel H7

N-(2-Methoxycarbonylphenyl-sulfonyl)-N'-[4-methoxy-6-(pyridin-2-ylthiomethyl)-pyrimidin-2-yl]-harnstoff (Verbindung Nr. 2.36)

7,0 g 2-Amino-4-methoxy-6-(pyridin-2-ylthiomethyl)-pyrimidin und 6,8 g 2-Methoxycarbonylphenylsulfonylisocyanat werden in 50 ml abs. Acetonitril für 2 Stunden bei 20-25 °C gerührt. Die trübe Lösung wird filtriert und das Lösungsmittel im Vakuum abgedampft. Der Rückstand wird aus Dioxan kristallisiert. Man erhält so 5,5 g (37 % d. Th.) N-(2-Methoxycarbonylphenyl-sulfonyl)-N'-[4-methoxy-6-(pyridin-2-ylthiomethyl)-pyrimidin-2-yl]-harnstoff, das mit 0,5 Mol Dioxan kristallisiert, Smp. 175-181 °C.

Beispiel H8

2-Amino-4-methoxy-6-äthoxythiocarbonylthiomethylpyrimidin (Verbindung Nr. 1.5)

16,0 g Kaliumäthylxanthogenat und 17,4 g 2-Amino-4-chlormethyl-6-methoxy-pyrimidin werden in 100 ml Aethanol für 30 min zum Rückfluss erhitzt. Nach dem Abkühlen der Reaktionslösung wird mit ca. 1 Liter Wasser verdünnt und das ausgefallene Produkt abgetrennt und getrocknet. Man erhält so 22,5 g (87 % d. Th.) 2-Amino-4-methoxy-6-äthoxythiocarbonylthiomethyl-pyrimidin, Smp. 91-93 °C.

Beispiel H9

2-Amino-4-methoxy-6-dimethylthiocarbamoylthiomethylpyrimidin (Verbindung Nr. 1.11)

18,5 g Dimethyldithiocarbamidsäure Natriumsalz Dihydrat und 17,4 g 2-Amino-4-chlormethyl-6-methoxy-pyrimidin werden in 100 ml Aethanol für 30 min zum Rückfluss erhitzt. Nach dem Abkühlen der Mischung wird mit ca. 1 Liter Wasser verdünnt und das ausgefallene Produkt abgetrennt und getrocknet. Man erhält so 23,0 g (89 % d. Th.) 2-Amino-4-methoxy-6-dimethylthiocarbamoylthiomethyl-pyrimidin, Smp. 118-120 °C.

Beispiel H10

2-Amino-4-methoxy-6-(4,5-dihydrothiazol-2-yl-thiomethyl)-pyrimidin (Verbindung Nr. 1.17)

11,9 g 4,5-Dihydro-2-mercapto-thiazol werden in 100 ml Methanol suspendiert. Dieser Suspension fügt man 18,0 g 30 %iger methanolischer Natriummethylatlösung zu. Es entsteht eine klare Lösung. Diese Lösung wird mit 17,4 g 2-Amino-4-chlormethyl-6-methoxy-pyrimidin versetzt und für 1 Stunde zum Rückfluss erhitzt. Nach dem Abkühlen der Lösung wird mit ca. 1 Liter Wasser verdünnt und das ausgefallene Produkt abgetrennt und getrocknet. Man erhält so 22,5 g (88 % d. Th.) 2-Amino-4-methoxy-6-(4,5-dihydrothiazol-2-yl-thiomethyl)-pyrimidin, Smp. 122-124 °C.

Beispiel H11

2-Amino-4-methoxy-6-(pyridin-2-yl-thiomethyl)-pyrimidin (Verbindung Nr. 1.18)

Eine Lösung von 11,1 g 2-Mercaptopyridin in 100 ml Methanol wird mit 18 g 30 %iger methanolischer Natriummethylatlösung versetzt und anschliessend für 1 Stunde bei 20-25 °C gerührt. In diese Lösung trägt man portionsweise 17,4 g 2-Amino-4-chlormethyl-6-methoxy-pyrimidin ein und rührt diese Mischung für weitere 2 Stunden bei 20-25 °C. Zur Aufarbeitung wird das Gemisch mit 1 Liter Wasser verdünnt und das ausfallende kristalline Produkt abgetrennt und getrocknet. Man erhält so 22,5 g (91 % d. Th.) 2-Amino-4-methoxy-6-(pyridin-2-ylthiomethyl)-pyrimidin, Smp. 115-116 °C.

Beispiel H12

2-Amino-4-methoxy-6-(di-n-propyloxythiophosphonylthiomethyl)-pyrimidin (Verbindung Nr. 1.32)

17,9 g 2-Amino-4-chlormethyl-6-methoxy-pyrimidin und 25,5 g Dithiophosphorsäure-O,O-di-n-propylester Kaliumsalz werden in 100 ml Aethylmethylketon für 1 Stunde zum Rückfluss erhitzt. Nach dem Abkühlen wird die Lösung mit 1 Liter Wasser verdünnt und mit 250 ml Aethylacetat extrahiert. Die organische Phase wird über Natriumsulfat getrocknet und eingedampft. Als Rückstand erhält man 34,0 g (97 % d. Th.) 2-Amino-4-methoxy-6-di-n-propyloxythiophosphonylthiomethylpyrimidin in Form eines farblosen viskosen Oeles.

In analoger Weise erhält man die zusätzlich in den anschliessenden Tabellen aufgeführten weiteren Zwischenprodukte und die Endprodukte der Formel I.

Tabelle 1

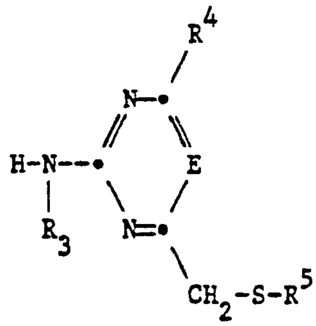

| Verb. Nr. | $R^3$ | E | $R^4$ | $R^5$ | physikalische Daten |
|---|---|---|---|---|---|
| 1.1 | H | CH | $OCH_3$ | $-CO-CH_3$ | hochviskos. Oel |
| 1.2 | H | N | $OCH_3$ | $-CO-CH_3$ | |
| 1.3 | H | CH | $OCH_3$ | $-CS-OCH_3$ | Smp. 97-100°C |
| 1.4 | H | N | $OCH_3$ | $-CS-OCH_3$ | |
| 1.5 | H | CH | $OCH_3$ | $-CS-OC_2H_5$ | Smp. 91-93°C |
| 1.6 | H | N | $OCH_3$ | $-CS-OC_2H_5$ | |
| 1.7 | $CH_3$ | CH | $OCH_3$ | $-CS-OC_2H_5$ | |
| 1.8 | $CH_3$ | N | $OCH_3$ | $-CS-OC_2-H_5$ | |
| 1.9 | H | CH | $OCH_3$ | $-CO-N(CH_3)_2$ | Smp. 155-157°C |
| 1.10 | H | N | $OCH_3$ | $-CO-N(CH_3)_2$ | |
| 1.11 | H | CH | $OCH_3$ | $-CS-N(CH_3)_2$ | Smp. 118-120°C |
| 1.12 | H | N | $OCH_3$ | $-CS-N(CH_3)_2$ | |
| 1.13 | H | CH | Cl | 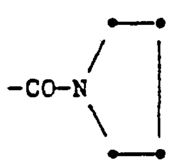 | Smp. 141-143°C |
| 1.14 | H | CH | $OCH_3$ | 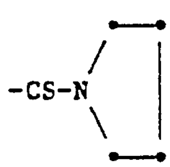 | Smp. 140-142°C |

Tabelle 1 (Fortsetzung)

| Verb. Nr. | $R^3$ | E | $R^4$ | $R^5$ | physikalische Daten |
|---|---|---|---|---|---|
| 1.15 | H | CH | $OCH_3$ | 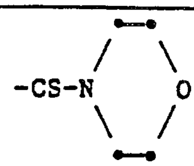 | Smp. 172-174°C |
| 1.16 | H | N | $OCH_3$ | 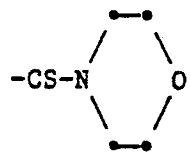 | |
| 1.17 | H | CH | $OCH_3$ | 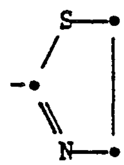 | Smp. 122-124°C |
| 1.18 | H | CH | $OCH_3$ | 2-Pyridyl | Smp. 115-116°C |
| 1.19 | H | N | $OCH_3$ | 2-Pyridyl | |
| 1.20 | $C_2H_5$ | N | $OCH_3$ | 2-Pyridyl | |
| 1.21 | $C_2H_5$ | CH | $OCH_3$ | 2-Pyridyl | |
| 1.22 | $C_2H_5$ | CH | $OCHF_2$ | 2-Pyridyl | |
| 1.23 | H | CH | $OCHF_2$ | 2-Pyridyl | |
| 1.24 | H | N | $OCHF_2$ | 2-Pyridyl | |
| 1.25 | H | CH | $CH_3$ | 2-Pyridyl | |
| 1.26 | H | CH | $OC_2H_5$ | 2-Pyridyl | |
| 1.27 | H | CH | $OCH_3$ | 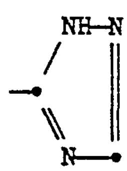 | Smp. 188-190°C |
| 1.28 | H | CH | $OCH_3$ | $-PS(OCH_3)_2$ | Oel |
| 1.29 | H | CH | $OCH_3$ | $-PS(OC_2H_5)_2$ | Oel |
| 1.30 | H | CH | $OCH_3$ | $-PO(OC_2H_5)_2$ | Oel |

Tabelle 1 (Fortsetzung)

| Verb. Nr. | $R^3$ | E | $R^4$ | $R^5$ | physikalische Daten |
|---|---|---|---|---|---|
| 1.31 | H | CH | $OCH_3$ | $-PS(OC_2H_4-OCH_3)_2$ | Oel |
| 1.32 | H | CH | $OCH_3$ | $-PS(OC_3H_7-n)_2$ | Oel |
| 1.33 | H | N | $CH_3$ | $-PS(OC_3H_7-n)_2$ | Oel |
| 1.34 | H | CH | $OCH_3$ | $-PS(OC_3H_7-i)_2$ | Oel |
| 1.35 | H | CH | $OCH_3$ | $-PS(OC_4H_9-n)_2$ | Oel |
| 1.36 | H | CH | $OCH_3$ | $-PO(OC_2H_5)-SC_3H_7-n$ | Oel |
| 1.37 | H | CH | $OCH_3$ | 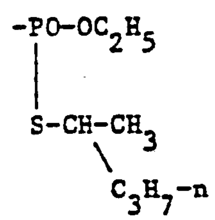 | Oel |

Tabelle 2

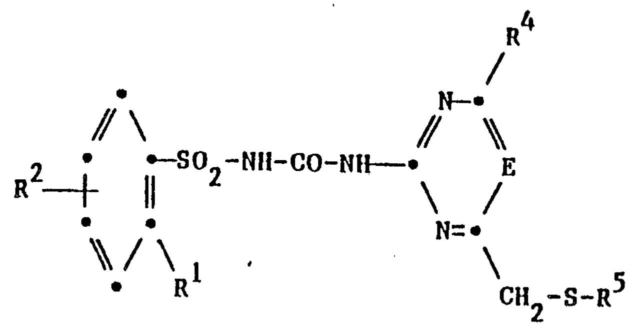

| Verb.Nr. | $R^1$ | $R^2$ | E | $R^4$ | $R^5$ | Smp.[°C] |
|---|---|---|---|---|---|---|
| 2.1 | $-COOCH_3$ | H | CH | $-OCH_3$ | $-CO-CH_3$ | 175-185 (Zers.) |
| 2.2 | $-COOCH_3$ | H | CH | $-OCH_3$ | $-CS-OCH_3$ | 148-151 |
| 2.3 | $-NO_2$ | H | CH | $-OCH_3$ | $-CS-OCH_3$ | 150-155 (Zers.) |
| 2.4 | $-COOCH_3$ | H | CH | $-OCH_3$ | $-CS-OC_2H_5$ | 160-162 (Zers.) |
| 2.5 | $-NO_2$ | H | CH | $-OCH_3$ | $-CS-OC_2H_5$ | 160-162 (Zers.) |
| 2.6 | $-COOCH_3$ | H | CH | $-OCH_3$ | $-CO-N(CH_3)_2$ | 175-177 (Zers.) |
| 2.7 | $-SO_2-N(CH_3)_2$ | H | CH | $-OCH_3$ | $-CS-N(CH_3)_2$ | 181-183 (Zers.) |
| 2.8 | $-CF_3$ | H | CH | $-OCH_3$ | $-CS-N(CH_3)_2$ | 202-204 (Zers.) |
| 2.9 | $-COOCH_3$ | H | CH | $-OCH_3$ | $-CS-N(CH_3)_2$ | 185-187 (Zers.) |
| 2.10 | $-NO_2$ | H | CH | $-OCH_3$ | $-CS-N(CH_3)_2$ | 202-204 (Zers.) |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.11 | $-COOCH_3$ | H | CH | $-OCH_3$ | 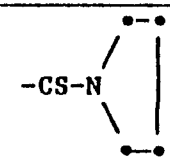 | 197-199 (Zers.) |
| 2.12 | $-CF_3$ | H | CH | $-OCH_3$ | 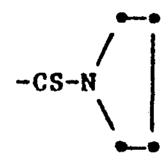 | 217-219 (Zers.) |
| 2.13 | $-COOCH_3$ | H | CH | $-OCH_3$ | 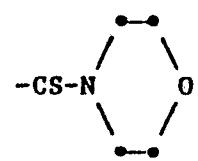 | 185-187 (Zers.) |
| 2.14 | $-COOCH_3$ | H | CH | -Cl | 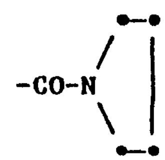 | 175-177 (Zers.) |
| 2.15 | $-COOCH_3$ | H | CH | $-OCH_3$ | 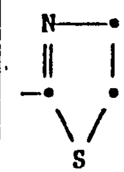 | 135-145 (Zers.) |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.16 | $-NO_2$ | H | CH | $-OCH_3$ | 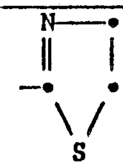 | 160-163 (Zers.) |
| 2.17 | $-OCHF_2$ | H | CH | -Cl | 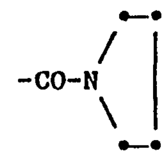 | 150-155 (Zers) |
| 2.18 | -Cl | H | CH | -Cl | 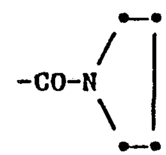 | 139-142 (Zers.) |
| 2.19 | $-NO_2$ | H | CH | -Cl | 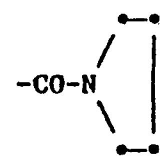 | 145-160 (Zers.) |
| 2.20 | $-OCHF_2$ | H | CH | $-OCH_3$ | 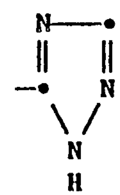 | 99-101 (Zers.) |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.21 | $-NO_2$ | H | N | $-CH_3$ | 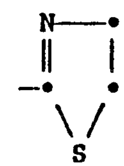 | |
| 2.22 | $-COOCH_3$ | H | CH | $-OCH_3$ | 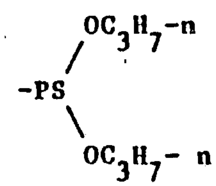 | 98-101 |
| 2.23 | $-NO_2$ | H | CH | $-OCH_3$ | 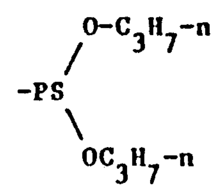 | 157-159 |
| 2.24 | $-COOCH_3$ | H | CH | $-OCH_3$ | 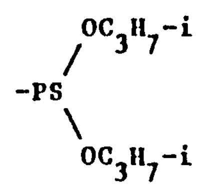 | 119-123 |
| 2.25 | $NO_2$ | H | CH | $-OCH_3$ | 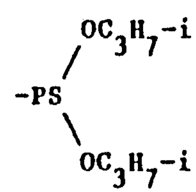 | 182-184 (Zers.) |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.26 | $-COOCH_3$ | H | CH | $-OCH_3$ | 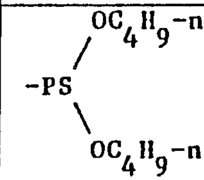 | 106-108 |
| 2.27 | $-NO_2$ | H | CH | $-OCH_3$ | 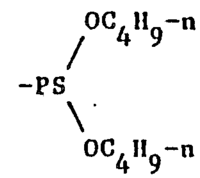 | 112-114 |
| 2.28 | -Cl | 3-Cl | CH | $-OCH_3$ | $-CS-N(CH_3)_2$ | 204-206 (Zers.) |
| 2.29 | -Cl | H | CH | $-OCH_3$ | $-PS(OC_2H_5)_2$ | 132-135 |
| 2.30 | -Cl | 3-Cl | CH | $-OCH_3$ | 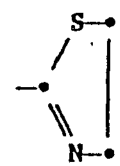 | 168-169 (Zers.) |
| 2.31 | -Cl | 6-Cl | CH | $-OCH_3$ | 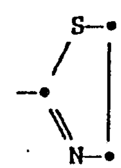 | 182-184 (Zers.) |
| 2.32 | -Cl | 3-Cl | CH | $-OCH_3$ | $-PS(OC_2H_5)_2$ | 136-138 |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.33 | $-CF_3$ | H | CH | $-OCH_3$ | 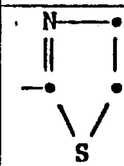 | 145-148 |
| 2.34 | $-OCH_3$ | H | CH | $-OCH_3$ | 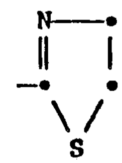 | 162-164 (Zers.) |
| 2.35 | $-OCH_3$ | H | CH | $-OCH_3$ | 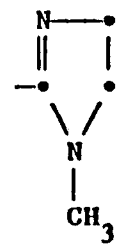 | 90-92 (Zers.) |
| 2.36 | $-COOCH_3$ | H | CH | $-OCH_3$ | 2-Pyridinyl | 182-184 (Zers.) |
| 2.37 | $-OCH_3$ | H | CH | $-OCH_3$ | 2-Pyrimidinyl | 197-199 (Zers.) |
| 2.38 | $-COOCH_3$ | H | CH | $-OCH_3$ | $-PS(OCH_3)_2$ | 152-154 (Zers.) |
| 2.39 | $-NO_2$ | H | CH | $-OCH_3$ | $-PS(OCH_3)_2$ | 120-125 (Zers.) |
| 2.40 | $-COOCH_3$ | H | CH | $-OCH_3$ | $-PS(OC_2H_5)_2$ | 128-130 |
| 2.41 | $-NO_2$ | H | CH | $-OCH_3$ | $-PS(OC_2H_5)_2$ | 168-170 |

Tabelle 2 (Fortsetzung)

| Verb.Nr. | $R^1$ | $R^2$ | E | $R^4$ | $R^5$ | Smp. [°C] |
|---|---|---|---|---|---|---|
| 2.42 | $-COOCH_3$ | H | CH | $-OCH_3$ | 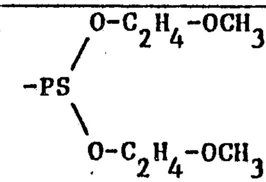 | 69-73 |
| 2.43 | $-NO_2$ | H | CH | $-OCH_3$ | 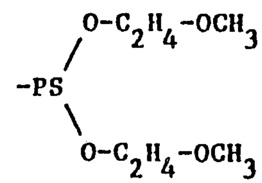 | 117-120 |
| 2.44 | $-NO_2$ | H | CH | $-OCH_3$ | $-PO-(OC_2H_5)_2$ | 154-159 |
| 2.45 | $-NO_2$ | H | CH | $-OCH_3$ | 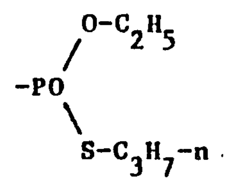 | 136-139 (Zers.) |
| 2.46 | $-COOCH_3$ | H | CH | $-OCH_3$ | 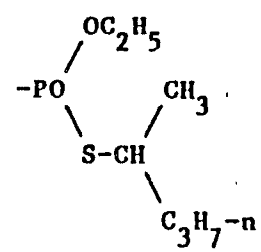 | Oel |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.47 | $-NO_2$ | H | CH | $-OCH_3$ | 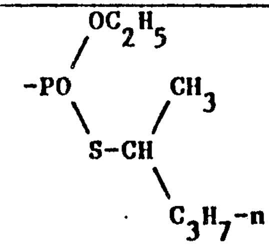 | Oel |
| 2.48 | $-OCH_3$ | H | CH | $-OCH_3$ | $-CS-OC_2H_5$ | |
| 2.49 | $-CF_3$ | H | N | $-OCH_3$ | 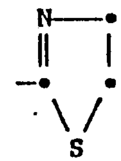 | |
| 2.50 | $-OCH_3$ | H | N | $-OCH_3$ | 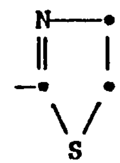 | |
| 2.51 | $-OCH_3$ | H | N | $-OCH_3$ | 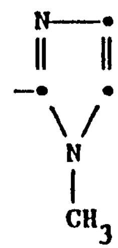 | |
| 2.52 | $-COOCH_3$ | H | N | $-OCH_3$ | 2-Pyridinyl | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.53 | $-OCH_3$ | H | N | $-OCH_3$ | 2-Pyrimidinyl | |
| 2.54 | $-COOCH_3$ | H | N | $-OCH_3$ | $-PS(OCH_3)_2$ | |
| 2.55 | $-NO_2$ | H | N | $-OCH_3$ | $-PS(OCH_3)_2$ | |
| 2.56 | $-COOCH_3$ | H | N | $-OCH_3$ | $-PS(OC_2H_3)_2$ | 160-163 (Zers.) |
| 2.57 | $-NO_2$ | H | N | $-OCH_3$ | $-PS(OC_2H_5)_2$ | |
| 2.58 | $-COOCH_3$ | H | N | $-OCH_3$ | $O-C_2H_4-OCH_3$<br>$-PS$<br>$O-C_2H_4-OCH_3$ | |
| 2.59 | $-NO_2$ | H | N | $-OCH_3$ | $O-C_2H_4-OCH_3$<br>$-PS$<br>$O-C_2H_4-OCH_3$ | |
| 2.60 | $-NO_2$ | H | N | $-OCH_3$ | $-PO(OC_2H_5)_2$ | |
| 2.61 | $-NO_2$ | H | N | -OCH3 | $O-C_2H_5$<br>$-PO$<br>$S-C_3H_7-n$ | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.62 | $-COOCH_3$ | H | N | $-OCH_3$ | 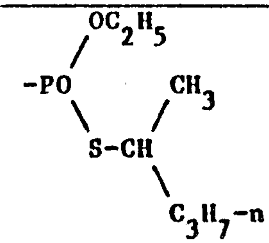 | |
| 2.63 | $-NO_2$ | H | N | $-OCH_3$ | 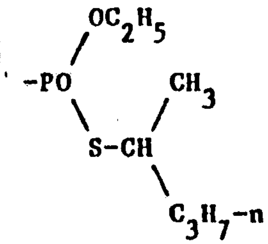 | |
| 2.64 | $-OCH_3$ | $-OCH_3$ | N | $-OCH_3$ | $-CS-OC_2H_5$ | |
| 2.65 | $-OCHF_2$ | H | N | -Cl | 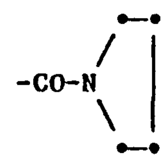 | |
| 2.66 | -Cl | H | N | -Cl | 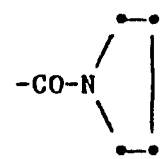 | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.67 | $-NO_2$ | H | N | -Cl | 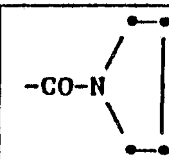 | |
| 2.68 | $-OCHF_2$ | H | N | $-OCH_3$ | 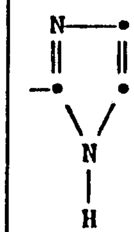 | |
| 2.69 | $-COOCH_3$ | H | N | $-OCH_3$ | 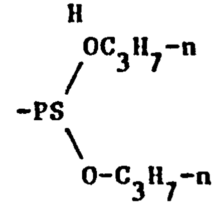 | |
| 2.70 | $-NO_2$ | H | N | $-OCH_3$ | 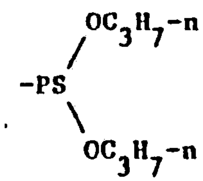 | |
| 2.71 | $-COOCH_3$ | H | N | $-OCH_3$ | 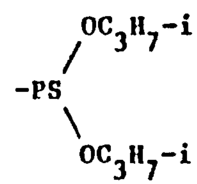 | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.72 | $-NO_2$ | H | N | $-OCH_3$ | 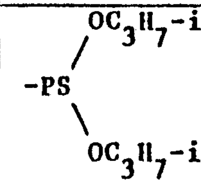 | |
| 2.73 | $-COOCH_3$ | H | N | $-OCH_3$ | 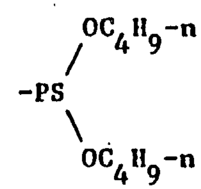 | |
| 2.74 | $-NO_2$ | H | N | $-OCH_3$ | 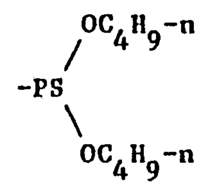 | |
| 2.75 | -Cl | 3-Cl | N | $-OCH_3$ | $-CS-N(CH_3)_2$ | |
| 2.76 | -Cl | H | N | $-OCH_3$ | $-PS(OC_2H_5)_2$ | |
| 2.77 | -Cl | 3-Cl | N | $-OCH_3$ | 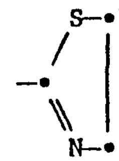 | |
| 2.78 | -Cl | 6-Cl | N | $-OCH_3$ | 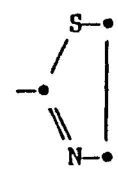 | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.79 | -Cl | 3-Cl | N | $-OCH_3$ | $-PS(OC_2H_5)_2$ | |
| 2.80 | $-COOCH_3$ | H | N | $-OCH_3$ | $-CO-CH_3$ | |
| 2.81 | $-COOCH_3$ | H | N | $-OCH_3$ | $-CS-OCH_3$ | |
| 2.82 | $-NO_2$ | H | N | $-OCH_3$ | $-CS-OCH_3$ | |
| 2.83 | $-COOCH_3$ | H | N | $-OCH_3$ | $-CS-OC_2H_5$ | |
| 2.84 | $-NO_2$ | H | N | $-OCH_3$ | $-CS-OC_2H_5$ | |
| 2.85 | $-COOCH_3$ | H | N | $-OCH_3$ | $-CO-N(CH_3)_2$ | |
| 2.86 | $-SO_2-N(CH_3)_2$ | H | N | $-OCH_3$ | $-CS-N(CH_3)_2$ | |
| 2.87 | $-CF_3$ | H | N | $-OCH_3$ | $-CS-N(CH_3)_2$ | |
| 2.88 | $-COOCH_3$ | H | N | $-OCH_3$ | $-CS-N(CH_3)_2$ | |
| 2.89 | $-NO_2$ | H | N | $-OCH_3$ | $-CS-N(CH_3)_2$ | |
| 2.90 | $-COOCH_3$ | H | N | $-OCH_3$ | -CS-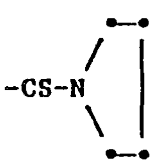 | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.91 | $-CF_3$ | H | N | $-OCH_3$ | 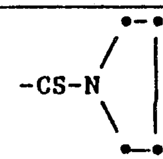 | |
| 2.92 | $-COOCH_3$ | H | N | $-OCH_3$ | 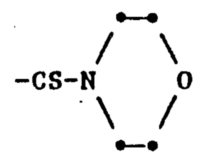 | |
| 2.93 | $-COOCH_3$ | H | N | -Cl | 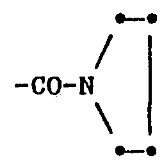 | |
| 2.94 | $-COOCH_3$ | H | N | $-OCH_3$ | 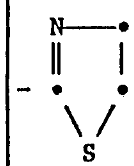 | |
| 2.95 | $-NO_2$ | H | N | $-OCH_3$ | 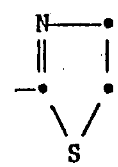 | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.96 | $-CF_3$ | H | N | $-CH_3$ | 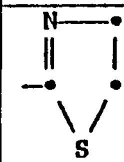 | |
| 2.97 | $-OCH_3$ | H | N | $-CH_3$ | 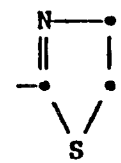 | |
| 2.98 | $-OCH_3$ | H | N | $-CH_3$ | 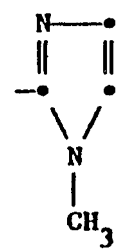 | |
| 2.99 | $-COOCH_3$ | H | N | $-CH_3$ | 2-Pyridinyl | |
| 2.100 | $-OCH_3$ | H | N | $-CH_3$ | 2-Pyrimidinyl | |
| 2.101 | $-COOCH_3$ | H | N | $-CH_3$ | $-PS(OCH_3)_2$ | |
| 2.102 | $-NO_2$ | H | N | $-CH_3$ | $-PS(OCH_3)_2$ | |
| 2.103 | $-COOCH_3$ | H | N | $-CH_3$ | $-PS(OC_2H_5)_2$ | |
| 2.104 | $-NO_2$ | H | N | $-CH_3$ | $-PS(OC_2H_5)_2$ | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.105 | $-COOCH_3$ | H | N | $-CH_3$ | 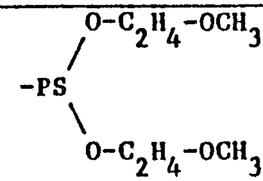 | |
| 2.106 | $-NO_2$ | H | N | $-CH_3$ | 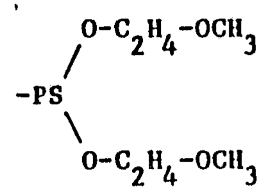 | |
| 2.107 | $-NO_2$ | H | N | $-CH_3$ | $-PO(OC_2H_5)_2$ | |
| 2.108 | $-NO_2$ | H | N | $-CH_3$ | 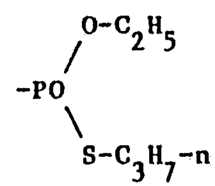 | |
| 2.109 | $-COOCH_3$ | H | N | $-CH_3$ | 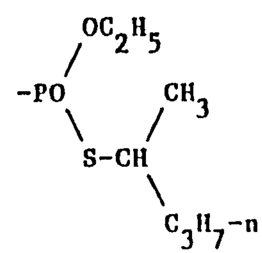 | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.110 | $-NO_2$ | H | N | $-CH_3$ | 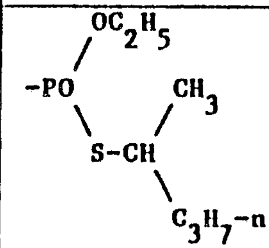 | |
| 2.111 | $OCH_3$ | $OCH_3$ | N | $-CH_3$ | $-CS-OC_2H_5$ | |
| 2.112 | $-OCHF_2$ | H | N | $-C_2H_5$ | 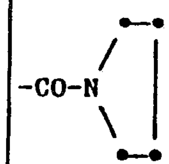 | |
| 1.113 | -Cl | H | N | $-C_2H_5$ | 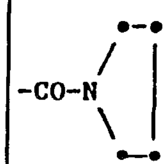 | |
| 2.114 | $-NO_2$ | H | N | $-C_2H_5$ | 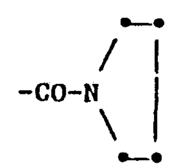 | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.115 | $-OCHF_2$ | H | N | $-CH_3$ | 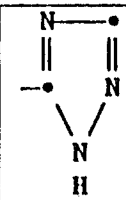 | |
| 2.116 | $-COOCH_3$ | H | N | $-CH_3$ | 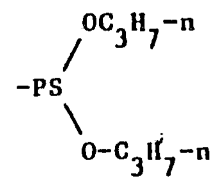 | |
| 2.117 | $-NO_2$ | H | N | $-CH_3$ | 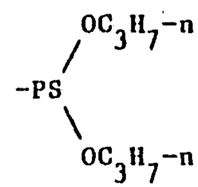 | |
| 2.118 | $-COOCH_3$ | H | N | $-CH_3$ | 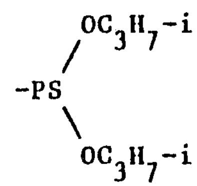 | |
| 2.119 | $-NO_2$ | H | N | $-CH_3$ | 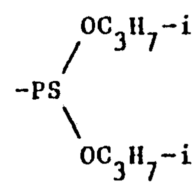 | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.120 | $COOCH_3$ | H | N | $-CH_3$ | 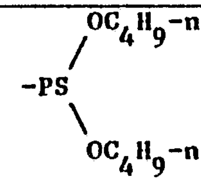 | |
| 2.121 | $-NO_2$ | H | N | $-CH_3$ | 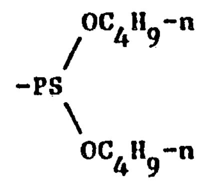 | |
| 2.122 | -Cl | 3-Cl | N | $-CH_3$ | $-CS-N(CH_3)_2$ | |
| 2.123 | -Cl | H | N | $-CH_3$ | $-PS(OC_2H_5)_2$ | |
| 2.124 | -Cl | 3-Cl | N | $-CH_3$ | 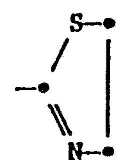 | |
| 2.125 | -Cl | 6-Cl | N | $-CH_3$ | 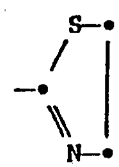 | |
| 2.126 | -Cl | 3-Cl | N | $-CH_3$ | $-PS(OC_2H_5)_2$ | |
| 2.127 | $-COOCH_3$ | H | N | $-CH_3$ | $-CO-CH_3$ | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.128 | $-COOCH_3$ | H | N | $-CH_3$ | $-CS-OCH_3$ | |
| 2.129 | $-NO_2$ | H | N | $-CH_3$ | $-CS-OCH_3$ | |
| 2.130 | $-COOCH_3$ | H | N | $-CH_3$ | $-CS-OC_2H_5$ | |
| 2.131 | $-NO_2$ | H | N | $-CH_3$ | $-CS-OC_2H_5$ | |
| 2.132 | $-COOCH_3$ | H | N | $-CH_3$ | $-CO-N(CH_3)_2$ | |
| 2.133 | $-SO_2-N(CH_3)_2$ | H | N | $-CH_3$ | $-CS-N(CH_3)_2$ | |
| 2.134 | $-CF_3$ | H | N | $-CH_3$ | $-CS-N(CH_3)_2$ | |
| 2.135 | $-COOCH_3$ | H | N | $-CH_3$ | $-CS-N(CH_3)_2$ | |
| 2.136 | $-NO_2$ | H | N | $-CH_3$ | $-CS-N(CH_3)_2$ | |
| 2.137 | $-COOCH_3$ | H | N | $-CH_3$ | -CS- 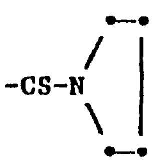 | |
| 2.138 | $-CF_3$ | H | N | $-CH_3$ | -CS- 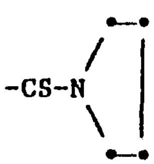 | |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.139 | $-COOCH_3$ | H | N | $-CH_3$ | 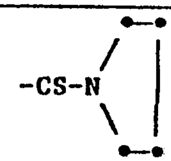 | |
| 2.140 | $-COOCH_3$ | H | N | $-OCHF_2$ | 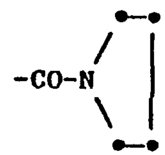 | |
| 2.141 | $-COOCH_3$ | H | N | $-CH_3$ | 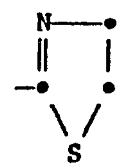 | |
| 2.142 | $-O-CCl=CHCl$ | H | CH | $-OCH_3$ | $-CS-N(CH_3)_2$ | 179-181 (Zers.) |
| 2.143 | $-OCHF_2$ | H | CH | Cl | $-CS-N(CH_3)_2$ | 171-173 (Zers.) |
| 2.144 | $-OCHF_2$ | H | CH | Cl | 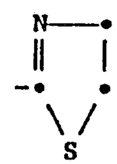 | 148-150 (Zers.) |

Tabelle 2 (Fortsetzung)

| Verb.Nr. | $R^1$ | $R^2$ | E | $R^4$ | $R^5$ | Smp.[°C] |
|---|---|---|---|---|---|---|
| 2.145 | $-O-SO_2CH_3$ | H | CH | $-OCH_3$ | $-CS-N(CH_3)_2$ | 173-177 (Zers.) |
| 2.146 | $-OCH_2CH_2Cl$ | H | CH | $-OCH_3$ | $-CS-N(CH_3)_2$ | 187-190 (Zers.) |
| 2.147 | $CF_3$ | H | CH | $-OCH_3$ | 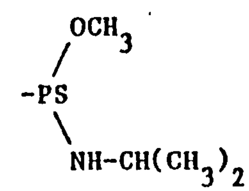 | 135-138 |
| 2.148 | Cl | H | N | $-OCH_3$ | $-CS-N(CH_3)_2$ | 167-169 (Zers.) |
| 2.149 | Cl | H | N | $-OCH_3$ | 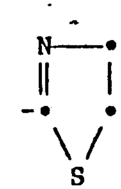 | 154-156 (Zers.) |

Tabelle 3

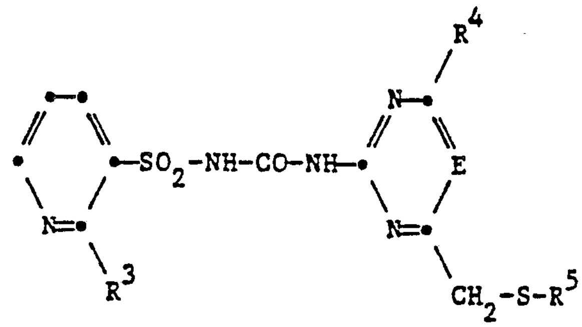

| Verb.Nr. | $R^3$ | E | $R^4$ | $R^5$ |
|---|---|---|---|---|
| 3.1 | Cl | CH | $OCH_3$ | $-CS-OCH_3$ |
| 3.2 | Cl | CH | $OCH_3$ | $-CS-OC_2H_5$ |
| 3.3 | Cl | CH | $OCH_3$ | $-CS-N(CH_3)2$ |
| 3.4 | Cl | CH | $OCH_3$ | $-CS-N(C_2H_5)_2$ |
| 3.5 | Cl | CH | $OCH_3$ | 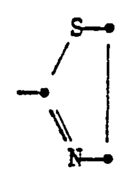 |
| 3.6 | Cl | CH | $OCH_3$ | $-PS(-OC_2H_5)_2$ |
| 3.7 | Cl | CH | $OCH_3$ | $-PO(OC_2H_5)_2$ |
| 3.8 | Cl | CH | $OCH_3$ | $-PS(OC_3H_7-n)_2$ |
| 3.9 | $CH_3$ | CH | Cl | $-CS-OC_2H_5$ |
| 3.10 | $CH_3$ | CH | $OC_2H_5$ | $-CS-OC_2H_5$ |
| 3.11 | $CH_3$ | CH | $CH_3$ | $-CS-OC_2H_5$ |
| 3.12 | $CH_3$ | CH | $OC_3H_7-i$ | $-CS-OC_2H_5$ |
| 3.13 | Cl | N | $OCH_3$ | $-CS-OCH_3$ |
| 3.14 | Cl | N | $CH_3$ | $-CS-OCH_3$ |
| 3.15 | $-COOCH_3$ | CH | $OCH_3$ | $-CS-OCH_3$ |
| 3.16 | $-COOCH_3$ | N | $CH_3$ | $-CS-OCH_3$ |

Tabelle 3 (Fortsetzung)

| Verb.Nr. | $R^3$ | E | $R^4$ | $R^5$ |
|---|---|---|---|---|
| 3.17 | $-COOCH_3$ | N | $OCH_3$ | $-CS-OCH_3$ |
| 3.18 | Cl | N | Cl | $-CS-OCH_3$ |
| 3.19 | Cl | N | $CH_3$ | $-CS-N(CH_3)_2$ |
| 3.20 | Cl | N | $CH_3$ | $-CS-OC_2H_5$ |
| 3.21 | Cl | N | $CH_3$ | $-PS(OC_2H_5)_2$ |
| 3.22 | Cl | N | $CH_3$ | $-PO(OCH_3)_2$ |
| 3.23 | Cl | N | $CH_3$ | $-PS(OC_3H_7-n)_2$ |
| 3.24 | Cl | N | $CH_3$ | 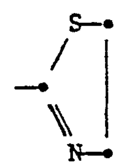 |

Tabelle 4

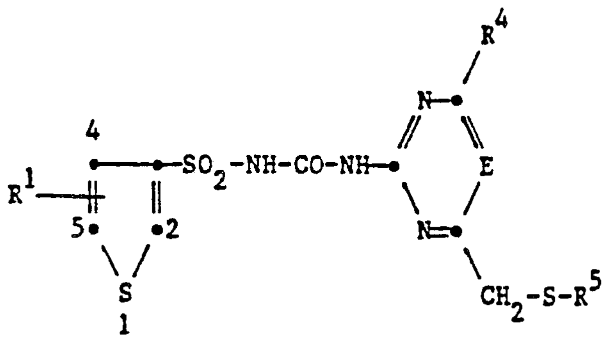

| Verb.Nr. | $R^1$ | E | $R^4$ | $R^5$ |
|---|---|---|---|---|
| 4.1 | $2-COOCH_3$ | CH | $OCH_3$ | $-CS-OCH_3$ |
| 4.2 | $2-COOCH_3$ | CH | $OCH_3$ | $-CS-OC_2H_5$ |
| 4.3 | $2-COOCH_3$ | CH | $OCH_3$ | $-CS-N(CH_3)_2$ |
| 4.4 | $2-COOCH_3$ | CH | $OCH_3$ | $-CS-N(C_2H_5)_2$ |
| 4.5 | $2-COOCH_3$ | CH | $OCH_3$ | 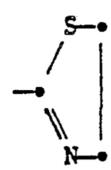 |
| 4.6 | $2-COOCH_3$ | CH | $OCH_3$ | $-PS(-OC_2H_5)_2$ |
| 4.7 | $2-COOCH_3$ | CH | $OCH_3$ | $-PO(OC_2H_5)_2$ |
| 4.8 | $2-COOCH_3$ | CH | $OCH_3$ | $-PS(OC_3H_7-n)_2$ |
| 4.9 | $2-COOCH_3$ | CH | Cl | $-CS-OC_2H_5$ |
| 4.10 | $2-COOCH_3$ | CH | $OC_2H_5$ | $-CS-OC_2H_5$ |
| 4.11 | $2-COOCH_3$ | CH | $CH_3$ | $-CS-OC_2H_5$ |
| 4.12 | $2-COOCH_3$ | CH | $OC_3H_7-i$ | $-CS-OC_2H_5$ |
| 4.13 | $2-COOCH_3$ | N | $OCH_3$ | $-CS-OCH_3$ |
| 4.14 | $2-COOCH_3$ | N | $CH_3$ | $-CS-OCH_3$ |
| 4.15 | $2-COOCH_3$ | CH | $OCH_3$ | $-CS-OCH_3$ |
| 4.16 | $2-COOCH_3$ | N | $CH_3$ | $-CS-OCH_3$ |

Tabelle 4 (Fortsetzung)

| Verb.Nr. | $R^1$ | E | $R^4$ | $R^5$ |
|---|---|---|---|---|
| 4.17 | $2-COOCH_3$ | N | $OCH_3$ | $-CS-OCH_3$ |
| 4.18 | $2-COOCH_3$ | N | Cl | $-CS-OCH_3$ |
| 4.19 | $2-COOCH_3$ | N | $CH_3$ | $-CS-N(CH_3)_2$ |
| 4.20 | $2-COOCH_3$ | N | $CH_3$ | $-CS-OC_2H_5$ |
| 4.21 | $2-COOCH_3$ | N | $CH_3$ | $-PS(OC_2H_5)_2$ |
| 4.22 | $2-COOCH_3$ | N | $CH_3$ | $-PO(OCH_3)_2$ |
| 4.23 | $2-COOCH_3$ | N | $CH_3$ | $-PS(OC_3H_7-n)_2$ |
| 4.24 | $2-COOCH_3$ | N | $CH_3$ | 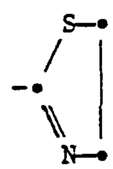 |
| 4.25 | $4-COOCH_3$ | CH | $OCH_3$ | $-CS-OCH_3$ |
| 4.26 | $4-COOCH_3$ | CH | $OCH_3$ | $-CS-OC_2H_5$ |
| 4.27 | $4-COOCH_3$ | CH | $OCH_3$ | $-CS-N(CH_3)_2$ |
| 4.28 | $4-COOCH_3$ | CH | $OCH_3$ | $-CS-N(C_3H_5)_2$ |
| 4.29 | $4-COOCH_3$ | CH | $OCH_3$ | 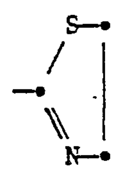 |
| 4.30 | $4-COOCH_3$ | CH | $OCH_3$ | $-PS(-OC_2H_5)_2$ |
| 4.31 | $4-COOCH_3$ | CH | $OCH_3$ | $-PO(OC_2H_5)_2$ |
| 4.32 | $4-COOCH_3$ | CH | $OCH_3$ | $-PS(OC_3H_7-n)_2$ |
| 4.33 | $4-COOCH_3$ | CH | Cl | $-CS-OC_2H_5$ |
| 4.34 | $3-COOCH_3$ | CH | $OC_2H_5$ | $-CS-OC_2H_5$ |
| 4.35 | $4-COOCH_3$ | CH | $CH_3$ | $-CS-OC_2H_5$ |
| 4.36 | $4-COOCH_3$ | CH | $OC_3H_7-i$ | $-CS-OC_2H_5$ |

Tabelle 4 (Fortsetzung)

| Verb.Nr. | $R^1$ | E | $R^4$ | $R^5$ |
|---|---|---|---|---|
| 4.37 | 4-$COOCH_3$ | N | $OCH_3$ | -CS-$OCH_3$ |
| 4.38 | 4-$COOCH_3$ | N | $CH_3$ | -CS-$OCH_3$ |
| 4.39 | 4-$COOCH_3$ | CH | $OCH_3$ | -CS-$OCH_3$ |
| 4.40 | 4-$COOCH_3$ | N | $CH_3$ | -CS-$OCH_3$ |
| 4.41 | 4-$COOCH_3$ | N | $OCH_3$ | -CS-$OCH_3$ |
| 4.42 | 4-$COOCH_3$ | N | Cl | -CS-$OCH_3$ |
| 4.43 | 4-$COOCH_3$ | N | $CH_3$ | -CS-$N(CH_3)_2$ |
| 4.44 | 4-$COOCH_3$ | N | $CH_3$ | -CS-$OC_2H_5$ |
| 4.45 | 4-$COOCH_3$ | N | $CH_3$ | -$PS(OC_2H_5)_2$ |
| 4.46 | 4-$COOCH_3$ | N | $CH_3$ | -$PO(OCH_3)_2$ |
| 4.47 | 4-$COOCH_3$ | N | $CH_3$ | -$PS(OC_3H_7$-n$)_2$ |
| 4.48 | 4-$COOCH_3$ | N | $CH_3$ | 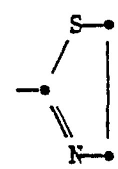 |

Formulierungsbeispiele

Beispiel F1 : Formulierungsbeispiele für Wirkstoffe der Formel I (% = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | - | - |
| Na-Diisobutylnaphthalinsulfonat | - | 6% | 6% |
| Octylphenolpolyäthylenglykol-äther (7-8 Mol AeO) | - | 2% | 2% |
| Hochdisperse Kieselsäure | 5% | 27% | 27% |
| Kaolin | 67% | - | - |
| Natriumchlorid | - | - | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff | 10% | 1% |
| Octylphenolpolyäthylenglykoläther (4-5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1% | 1% |
| Talkum | 99,9% | - |
| Kaolin | - | 99% |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40% | 5% |
| Aethylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff | 5% |
| Isopropylamin | 1% |
| Octylphenolpolyäthylenglykoläther (78 Mol AeO) | 3% |
| Wasser | 91% |

Biologische Beispiele

Beispiel B1 : Herbizidwirkung vor dem Auflaufen der Pflanzen

Kunststofftöpfe werden mit expandiertem Vermiculit (Dichte : 0,135 g/cm$^3$, Wasserabsorptionsvermögen : 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät : Nasturtium officinalis, Agrostis tenuis, Stellaria media und Digitaria sanguinalis. Die Versuchsgefässe werden anschliessend in einer Klimakammer bei 20 °C, einer Beleuchtung von ca. 20 klux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5 % eines handelsüblichen Flüssigdüngers (®Greenzit, ex Ciba-Geigy) zugesetzt. 12 Tage nach der Aussaat wird der Versuch ausgewertet und die Wirkung auf die Versuchspflanzen nach dem folgenden Massstab bewertet :

1 : Pflanze nicht gekeimt
2-3 : sehr starke Wirkung
4-6 : mittlere Wirkung
7-8 : schwache Wirkung
9 : keine Wirkung (wie unbehandelte Kontrolle).

pre-emergente Wirkung :

Konzentration der Wirkstoffemulsion : 70.8 ppm

| Testpflanze / Wirkstoff Nr. | Nasturtium | Stellaria | Agrostis | Digitaria |
|---|---|---|---|---|
| 2.1 | 2 | 2 | 1 | 1 |
| 2.2 | 1 | 1 | 1 | 1 |
| 2.3 | 2 | 1 | 1 | 2 |
| 2.4 | 1 | 2 | 1 | 2 |
| 2.5 | 2 | 1 | 1 | 1 |
| 2.6 | 2 | 1 | 2 | 2 |
| 2.7 | 2 | 1 | 1 | 2 |
| 2.8 | 1 | 2 | 1 | 2 |
| 2.9 | 2 | 1 | 2 | 2 |
| 2.10 | 2 | 1 | 1 | 1 |
| 2.11 | 2 | 2 | 1 | 2 |
| 2.13 | 2 | 1 | 1 | 2 |
| 2.14 | 2 | 2 | 2 | 3 |
| 2.15 | 1 | 2 | 1 | 2 |
| 2.16 | 1 | 1 | 1 | 1 |
| 2.17 | 3 | 2 | 2 | 5 |

(Fortsetzung)

| Testpflanze / Wirkstoff Nr. | Nasturtium | Stellaria | Agrostis | Digitaria |
|---|---|---|---|---|
| 2.19 | 1 | 1 | 1 | 2 |
| 2.20 | 1 | 1 | 1 | 2 |
| 2.22 | 2 | 1 | 1 | 1 |
| 2.23 | 2 | 2 | 1 | 2 |
| 2.24 | 2 | 1 | 2 | 2 |
| 2.25 | 2 | 2 | 1 | 2 |
| 2.26 | 1 | 1 | 1 | 1 |
| 2.27 | 1 | 1 | 1 | 2 |
| 2.28 | 2 | 2 | 2 | 3 |
| 2.29 | 1 | 2 | 1 | 2 |
| 2.30 | 1 | 1 | 1 | 2 |
| 2.31 | 1 | 1 | 1 | 2 |
| 2.32 | 1 | 2 | 1 | 3 |
| 2.33 | 2 | 2 | 2 | 2 |
| 2.34 | 1 | 1 | 1 | 2 |
| 2.35 | 2 | 2 | 2 | 2 |
| 2.36 | 2 | 1 | 1 | 2 |
| 2.37 | 1 | 1 | 1 | 2 |
| 2.38 | 1 | 1 | 1 | 1 |
| 2.39 | 1 | 2 | 1 | 2 |
| 2.40 | 2 | 1 | 1 | 2 |
| 2.41 | 1 | 2 | 1 | 2 |
| 2.42 | 2 | 1 | 1 | 1 |
| 2.43 | 1 | 2 | 1 | 2 |
| 2.44 | 1 | 1 | 1 | 1 |
| 2.45 | 2 | 1 | 1 | 2 |
| 2.46 | 2 | 1 | 1 | 2 |
| 2.47 | 2 | 2 | 1 | 2 |
| 2.142 | 2 | 2 | 2 | 2 |
| 2.145 | 2 | 1 | 2 | 1 |
| 2.146 | 2 | 2 | 2 | 2 |
| 2.147 | 1 | 2 | 1 | 2 |
| 2.148 | 2 | 2 | 2 | 2 |
| 2.149 | 2 | 2 | 2 | 2 |

Beispiel B2 : Wuchshemmung bei tropischen Bodenbedecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6 000 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit der Wirkstoffen der Formel I behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel B3 : Wuchsregulierung an Sojabohnen

In Kunststoffbehältern mit einem Erde-Torf-Gemisch im Verhältnis 6 : 3 : 1 werden Sojabohnen der Sorte « Hark » angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

Beispiel B4 : Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel B5 : Wuchshemmung bei Gräsern

In Kunststoffschalen mit Erde-Torf-Sand-Gemisch (6 : 3 : 1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffs der Formel I besprüht. Die Wirkstoffmenge beträgt bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses um 10-30 % im Vergleich zur unbehandelten Kontrolle.

**Patentansprüche**

1. N-Arylsulfonyl-N'-(4-mercaptomethyl-pyrimidinyl- und -triazinyl)-harnstoffe der Formel I

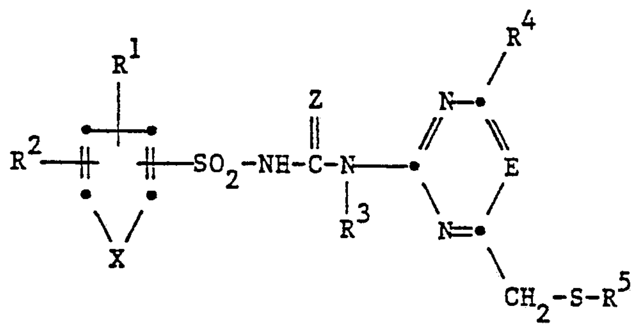

(I)

worin

E Stickstoff oder —CH=,

X Sauerstoff, Schwefel, $—NR^3—$, $—N{=}CR^3—$, —CH=CH— oder

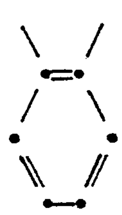

Z Sauerstoff oder Schwefel,

$R^1$ Wasserstoff, Halogen, Nitro, Aethinyl, $—NR^{16}R^{17}$, $—CR^6$-di-$C_1$-$C_4$-Alkoxy,

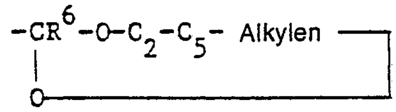

—CW—$R^6$, —$SO_2$—$NR^7R^8$, —CO—$R^9$, —$Y_m$—$R^{10}$, —$SO_2$—$R^{11}$ oder —O—$SO_2R^{12}$, wobei m für Null oder eins steht,

$R^2$ Wasserstoff, Halogen, $C_1$-$C_4$-Alkyl, $C_1$-$C_4$-Alkoxy, $C_1$-$C_4$-Halogenalkyl oder Nitro,

$R^3$ Wasserstoff, $C_1$-$C_4$-Alkyl, $C_3$-$C_4$-Alkenyl oder $C_1$-$C_4$-Alkoxy,

$R^4$ Wasserstoff, Halogen, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkylthio, $C_2$-$C_4$-Alkoxyalkyl, $C_2$-$C_4$-Alkoxyalkoxy, Cyclopropyl, —$NH_2$, $C_1$-$C_4$-Alkylamino, Di-$C_1$-$C_4$-alkylamino oder einen über das Stickstoffatom gebundenen gesättigten 5- bis 7-gliedrigen Stickstoffheterocyclus, der noch ein weiteres Heteroatom enthalten kann,

$R^5$ Cyan, —CZ—$R^{13}$,

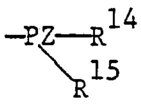

oder einen unsubstituierten oder einen durch einen Rest aus der Gruppe Halogen, $C_1$-$C_4$-Alkyl, $C_1$-$C_4$-Alkoxy oder $C_1$-$C_4$-Halogenalkyl substituierten ungesättigten Heterocyclus ausgewählt aus Reihe Imidazol, Triazol, Pyridin, Pyrimidin, Thiazol, Oxazol, Thiadiazol, Oxadiazol, Pyridazin, Thiophen oder Furan oder deren teilhydrierten Abkömmlingen,

$R^6$ Wasserstoff, $C_1$-$C_4$-Alkyl, $C_1$-$C_4$-Halogenalkyl, $C_3$-$C_6$-Cycloalkyl, $C_4$-$C_7$-Cycloalkylalkyl oder $C_2$-$C_4$-Alkoxyalkyl,

$R^7$ und $R^{16}$ unabhängig voneinander Wasserstoff, $C_1$-$C_4$-Alkyl $C_1$-$C_4$-Cyanoalkyl oder $C_1$-$C_4$-Alkoxy,

$R^8$ und $R^{17}$ unabhängig voneinander Wasserstoff, $C_1$-$C_4$-Alkyl, $C_3$-$C_4$-Alkenyl oder $C_1$-$C_4$-Alkoxy, oder

$R^7$ und $R^8$ sowie $R^{16}$ und $R^{17}$ unabhängig voneinander zusammen mit dem sie bindenden Stickstoffatom einen 5- bis 7-gliedrigen gesättigten Stickstoffheterocyclus, der noch ein weiteres Heteroatom enthalten kann,

$R^9$ $C_1$-$C_4$-Alkoxy, $C_3$-$C_6$-Alkenyloxy, $C_3$-$C_6$-Alkinyloxy, $C_2$-$C_6$-Halogenalkoxy, $C_1$-$C_4$-Cyanalkoxy, $C_1$-$C_4$-Alkylthio. $C_3$-$C_4$-Alkenylthio, $C_3$-$C_4$-Alkinylthio, $C_5$-$C_6$-Cycloalkoxy, $C_4$-$C_7$-Cycloalkylalkoxy, —$NR^7R^8$ oder $C_2$-$C_6$-Alkoxyalkoxy,

$R^{10}$ $C_3$-$C_4$-Alkinyl, $C_2$-$C_4$-Alkenyl, $C_1$-$C_4$-Alkyl, ein- oder mehrfach durch Halogen, Cyan, $C_1$-$C_4$-Alkoxy oder —$SO_n$-$C_1$-$C_4$-Alkyl substituiertes $C_2$-$C_4$-Alkenyl oder ein- oder mehrfach durch Halogen, Cyan, Nitro, $C_1$-$C_4$-Alkoxy, $C_1$-$C_4$-Halogenalkoxy, $C_1$-$C_4$-Halogenalkylthio, —$SO_n$-$C_1$-$C_4$-Alkyl, —T—CX—$R^{18}$,

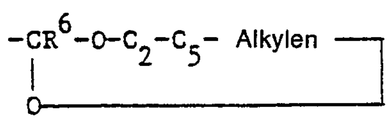

—CO—$R^6$, —CO—$R^9$, oder —$SO_2$—$NR^7R^8$ substituiertes $C_1$-$C_4$-Alkyl, wobei n für Null, 1 oder 2 steht,

$R^{11}$ $C_2$-$C_4$-Halogenalkoxy,

$R^{12}$ $C_1$-$C_4$-Alkyl, $C_1$-$C_4$-Halogenalkyl oder —$NR^{16}R^{17}$,

$R^{13}$ $C_1$-$C_4$-Alkyl, $C_1$-$C_4$-Alkoxy, $C_1$-$C_4$-Alkylthio, Phenyl, Di-$C_1$-$C_4$-alkylamino oder über das Stickstoffatom gebundenen gesättigten 5- bis 7-gliedrigen Stickstoffheterocyclus, der noch ein weiteres Heteroatom enthalten kann,

$R^{14}$ und $R^{15}$ unabhängig voneinander $C_1$-$C_4$-Alkyl, $C_1$-$C_4$-Alkoxy, $C_3$-$C_4$-Alkoxyalkoxy, $C_3$-$C_4$-Alkenyloxy, $C_1$-$C_6$-Alkylthio, $C_1$-$C_4$-Alkylamino oder Di-$C_1$-$C_4$-alkylamino,

$R^{18}$ $C_1$-$C_4$-Alkyl, $C_1$-$C_4$-Halogenalkyl, $C_1$-$C_4$-Alkoxy, $C_1$-$C_4$-Alkylthio oder —$NR^{16}R^{17}$,

T Sauerstoff oder Schwefel,

W Sauerstoff oder =N—O—$R^3$ und

Y Sauerstoff, Schwefel, —SO— oder —$SO_2$

bedeuten, sowie die Salze dieser Verbindungen.

2. Verbindungen gemäss Anspruch 1, dadurch gekennzeichnet, dass X für —CH=CH— steht.

3. Verbindungen gemäss Anspruch 1, dadurch gekennzeichnet, dass Z für Sauerstoff steht.

4. Verbindungen gemäss Anspruch 1, dadurch gekennzeichnet, dass $R^1$ für $C_1$-$C_4$-Alkoxycarbonyl, Nitro, Halogen, $C_1$-$C_4$-Halogenalkyl, $C_1$-$C_4$-Halogenalkoxy oder Di-$C_1$-$C_4$-alkylsulfamoyl steht.

5. Verbindungen gemäss Anspruch 1, dadurch gekennzeichnet, dass $R^2$ Wasserstoff ist.

6. Verbindungen gemäss Anspruch 1, dadurch gekennzeichnet, dass $R^3$ Wasserstoff ist.

7. Verbindungen gemäss Anspruch 1, dadurch gekennzeichnet, dass $R^4$ für Halogen, $C_1$-$C_4$-Alkoxy oder $C_1$-$C_4$-Alkyl steht.

8. Verbindungen gemäss Anspruch 1, dadurch gekennzeichnet, dass E für die Methinbrücke steht.

9. Verbindungen gemäss Anspruch 1, dadurch gekennzeichnet, dass $R^5$ für $C_1$-$C_4$-Alkylcarbonyl, $C_1$-$C_4$-Alkoxycarbonyl, $C_1$-$C_4$-Alkoxythiocarbonyl, Di-$C_1$-$C_4$-alkylcarbamoyl, Di-$C_1$-$C_4$-alkylthiocarbamoyl, Di-$C_1$-$C_4$-alkoxyphosphonyl, Di-$C_1$-$C_4$-alkoxythiophosphonyl, N-Pyrrolidinocarbonyl, N-Pyrrolidinothiocarbonyl, N-Morpholinothiocarbonyl, 2H-1,3,4-Triazol-3-yl, 4,5-Dihydrothiazol-2-yl, 1-$C_1$-$C_4$-Alkyl-imidazol-2-yl, 2-Pyridinyl oder 2-Pyrimidinyl steht.

10. Verbindungen gemäss Anspruch 1, dadurch gekennzeichnet, dass X die Aethenylenbrücke, Z Sauerstoff, $R^1$ $C_1$-$C_4$-Alkoxycarbonyl, Nitro, Halogen, $C_1$-$C_4$-Halogenalkyl, $C_1$-$C_4$-Halogenalkoxy oder Di-$C_1$-$C_4$-alkylsulfamoyl und $R^2$ und $R^3$ Wasserstoff bedeuten.

11. Verbindungen gemäss Anspruch 1, dadurch gekennzeichnet, dass $R^4$ Halogen, $C_1$-$C_4$-Alkoxy oder $C_1$-$C_4$-Alkyl, E die Methinbrücke und $R^5$ $C_1$-$C_4$-Alkylcarbonyl, $C_1$-$C_4$-Alkoxycarbonyl, $C_1$-$C_4$-Alkoxythiocarbonyl, Di-$C_1$-$C_4$-alkylcarbamoyl, Di-$C_1$-$C_4$-alkylthiocarbamoyl, Di-$C_1$-$C_4$-alkoxyphosphonyl, Di-$C_1$-$C_4$-alkoxythiophosphonyl, N-Pyrrolidinocarbonyl, N-Pyrrolidinothiocarbonyl, N-Morpholinothiocarbonyl, 1H-1,2,4-Triazol-3-yl, 4,5-Dihydrothiazol-2-yl, 1-$C_1$-$C_4$-Alkylimidazol-2-yl, 2-Pyridinyl oder 2-Pyrimidinyl bedeuten.

12. Verbindungen gemäss Anspruch 1, dadurch gekennzeichnet, dass X die Aethenylenbrücke, Z Sauerstoff, $R^1$ $C_1$-$C_4$-Alkoxycarbonyl, Nitro, Halogen, $C_1$-$C_4$-Halogenalkyl, $C_1$-$C_4$-Halogenalkoxy oder Di-$C_1$-$C_4$-alkylsulfamoyl und $R^2$ und $R^3$ Wasserstoff, Halogen, $C_1$-$C_4$-Alkoxy oder $C_1$-$C_4$-Alkyl, E die Methinbrücke und $R^5$ $C_1$-$C_4$-Alkylcarbonyl, $C_1$-$C_4$-Alkoxycarbonyl, $C_1$-$C_4$-Alkoxythiocarbonyl, Di-$C_1$-$C_4$-alkylcarbamoyl, Di-$C_1$-$C_4$-alkylthiocarbamoyl, Di-$C_1$-$C_4$-alkoxyphosphonyl, Di-$C_1$-$C_4$-alkoxythiophosphonyl, N-Pyrrolidinocarbonyl, N-Pyrrolidinothiocarbonyl, N-Morpholinothiocarbonyl, 2H-1,2,4-Triazol-3-yl, 4,5-Dihydrothiazol-2-yl, 1-$C_1$-$C_4$-Alkyl-imidazol-2-yl, 2-Pyridinyl oder 2-Pyrimidinyl bedeuten.

13. N-(2-Methoxycarbonylphenyl-sulfonyl)-N'-(4-methoxy-6-acetylthiomethyl-pyrimidin-2-yl)-harnstoff gemäss Anspruch 1.

14. N-(2-Methoxycarbonylphenyl-sulfonyl)-N'(4-methoxy-6-methoxythiocarbonylthiomethyl-pyrimidin-2-yl)-harnstoff gemäss Anspruch 1.

15. N-(2-Nitrophenyl-sulfonyl)-N'-[4-methoxy-6-(4,5-dihydrothiazol-2-ylthiomethyl)-pyrimidin-2-yl]-harnstoff gemäss Anspruch 1.

16. N-(2-Methoxycarbonylphenyl-sulfonyl)-N'-(4-methoxy-6-dimethoxythiophosphonylthiomethyl-pyrimidin-2-yl)-harnstoff gemäss Anspruch 1.

17. N-(2-Nitrophenyl-sulfonyl)-N'-(4-methoxy-6-diäthoxyphosphonylthiomethyl-pyrimidin-2-yl)-harnstoff gemäss Anspruch 1.

18. N-(2-Methoxycarbonylphenyl-sulfonyl)-N'-(4-methoxy-6-di-n-butyloxythiophosphonyl-thiomethyl-pyrimidin-2-yl)-harnstoff gemäss Anspruch 1.

19. Verfahren zur Herstellung der Verbindungen der Formel I gemäss Anspruch 1, dadurch gekennzeichnet, dass man ein Arylsulfonylisocyanat oder -isothiocyanat der Formel II

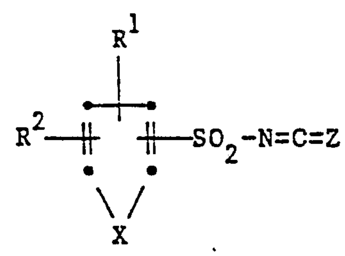

(II)

worin $R^1$, $R^2$, X und Z die unter Formel I gegebene Bedeutung haben, mit einem Aminopyrimidin oder -triazin der Formel III

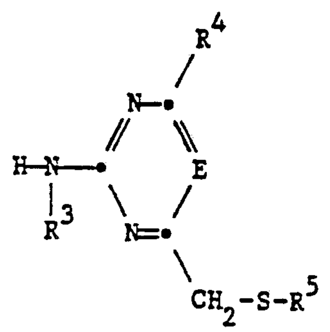

(III)

worin $R^3$, $R^4$, $R^5$ und E die unter Formel I gegebene Bedeutung haben, umsetzt und gegebenenfalls in die Salze überführt.

20. Verfahren zur Herstellung der Verbindungen der Formel I gemäss Anspruch 1, dadurch gekennzeichnet, dass man ein N-Arylsulfonylcarbamat der Formel IV

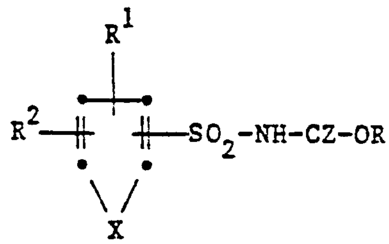

(IV)

worin $R^1$, $R^2$, X und Z die unter Formel I gegebene Bedeutung haben und R für Phenyl, Alkyl oder substituiertes Phenyl steht, mit einem Aminopyrimidin oder -triazin der Formel III umsetzt und gegebenenfalls in die Salze überführt.

21. Verfahren zur Herstellung der Verbindungen der Formel I, dadurch gekennzeichnet, dass man eine Verbindung der Formel V

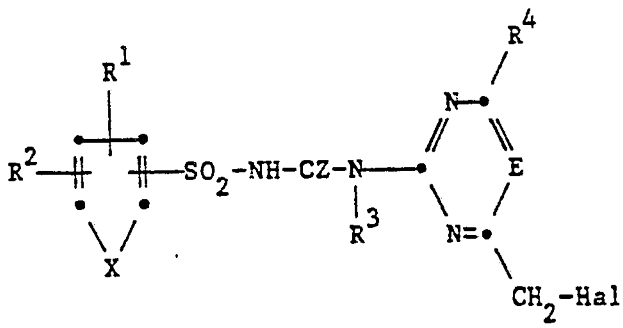

(V)

worin $R^1$, $R^2$, $R^3$, $R^4$, X, E und Z die unter Formel I gegebene Bedeutung haben und Hal für Chlor oder Brom steht, mit einem Schwefelsalz der Formel VI

$$M^{\oplus}S^{\ominus}—R^5 \qquad \text{(VI)},$$

worin $R^5$ die unter Formel I gegebene Bedeutung hat und $M^{\oplus}$ für ein Alkali-, Erdalkali- oder Ammoniumkation steht, umsetzt und gegebenenfalls in die Salze überführt.

22. Verfahren zur Herstellung von Additionssalzen der Formel I gemäss einem der Ansprüche 19 bis 21, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

23. Ein herbizides und den Pflanzenwuchs hemmendes Mittel, dadurch gekennzeichnet, dass es neben Träger- und/oder anderen Zuschlagstoffen als Wirkstoff mindestens einen Arylsulfonyl-N'-(4-mercaptomethylpyrimidinyl- oder -triazinyl)-harnstoff der Formel I, Anspruch 1, enthält.

24. Die Verwendung der Wirkstoffe der Formel I gemäss Anspruch 1, oder sie enthaltender Mittel zur Bekämpfung unerwünschten Pflanzenwachstums.

25. Die Verwendung der Wirkstoffe der Formel I gemäss Anspruch 1, oder sie enthaltender Mittel zur Hemmung des Pflanzenwachstums.

26. Die Verwendung der Wirkstoffe der Formel I, oder sie enthaltender Mittel gemäss Anspruch 25, zur selektiven pre- oder post-emergenten Bekämpfung von Unkräutern in Nutzpflanzenkulturen.

27. Verfahren zur selektiven Bekämpfung von Unkräutern in Nutzpflanzenkulturen, dadurch gekennzeichnet, dass man die Kulturen oder deren Anbaufläche mit einer wirksamen Menge eines Wirkstoffs der Formel I, Anspruch 1, behandelt.

28. Aminopyrimidine und -triazine der Formel III

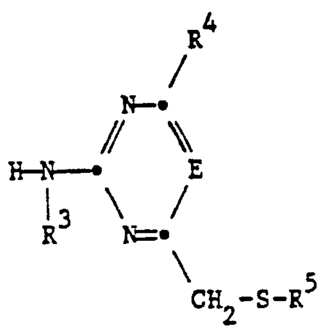

(III)

worin $R^3$, $R^4$, $R^5$ und E die unter Formel I im Anspruch 1 gegebene Bedeutung haben.

**Claims**

1. N-Arylsulfonyl-N'-(4-mercaptomethyl-pyrimidinyl- and -triazinyl)-ureas of the formula I

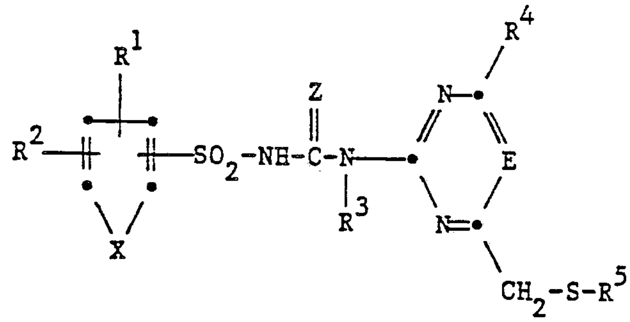

(I)

wherein
E is nitrogen or —CH=,
X is oxygen, sulfur, $—NR^3—$, $—N=CR^3—$, —CH=CH— or

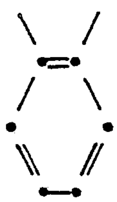

Z is oxygen or sulfur,
$R^1$ is hydrogen, halogen, nitro, ethinyl, $—NR^{16}R^{17}$, $—CR^6$-di-$C_1$-$C_4$-alkoxy,

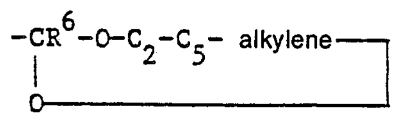

$—CW—R^6$, $—SO_2—NR^7R^8$, $—CO—R^9$, $Y_m—R^{10}$, $—SO_2—R^{11}$ or $—O—SO_2R^{12}$, in which m is zero or 1,
$R^2$ is hydrogen, halogen, $C_1$-$C_4$-alkyl, $C_1$-$C_4$-alkoxy, $C_1$-$C_4$-haloalkyl or nitro,
$R^3$ is hydrogen, $C_1$-$C_4$-alkyl, $C_3$-$C_4$-alkenyl or $C_1$-$C_4$-alkoxy,
$R^4$ is hydrogen, halogen, $C_1$-$C_4$-alkyl, $C_1$-$C_4$-alkoxy, $C_1$-$C_4$-haloalkyl, $C_1$-$C_4$-haloalkoxy, $C_1$-$C_4$-alkylthio, $C_2$-$C_4$-alkoxyalkyl, $C_2$-$C_4$-alkoxyalkoxy, cyclopropyl, $—NH_2$, $C_1$-$C_4$-alkylamino, di-$C_1$-$C_4$-alkylamino, or a saturated 5- to 7-membered nitrogen heterocycle which is bound by way of the nitrogen atom and which can contain a further hetero atom,
$R^5$ is cyano, $—CZ—R^{13}$,

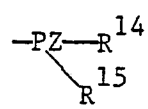

or an unsaturated heterocycle that is selected from the series comprising imidazole, triazole, pyridine, pyrimidine, thiazole, oxazole, thiadiazole, oxadiazole, pyridazine, thiophene or furan, or the partially hydrogenated derivatives thereof, and that is unsubstituted or substituted by a radical selected from the group comprising : halogen, $C_1$-$C_4$-alkyl, $C_1$-$C_4$-alkoxy or $C_1$-$C_4$-haloalkyl,
$R^6$ is hydrogen, $C_1$-$C_4$-alkyl, $C_1$-$C_4$-haloalkyl, $C_3$-$C_6$-cycloalkyl, $C_4$-$C_7$-cycloalkylalkyl or $C_2$-$C_4$-alkoxyalkyl,
$R^7$ and $R^{16}$ independently of one another are hydrogen, $C_1$-$C_4$-alkyl, $C_1$-$C_4$-cyanoalkyl or $C_1$-$C_4$-alkoxy,
$R^8$ and $R^{17}$ independently of one another are hydrogen, $C_1$-$C_4$-alkyl, $C_3$-$C_4$-alkenyl or $C_1$-$C_4$-alkoxy, or
$R^7$ and $R^8$ as well as $R^{16}$ and $R^{17}$ independently of one another form, together with the nitrogen atom binding them, a 5- to 7-membered saturated nitrogen heterocycle, which can contain a further hetero atom,
$R^9$ is $C_1$-$C_4$-alkoxy, $C_3$-$C_6$-alkenyloxy, $C_3$-$C_6$-alkinyloxy, $C_2$-$C_6$-haloalkoxy, $C_1$-$C_4$-cyanoalkoxy, $C_1$-

$C_4$-alkylthio, $C_3$-$C_4$-alkenylthio, $C_3$-$C_4$-alkinylthio, $C_5$-$C_6$-cycloalkoxy, $C_4$-$C_7$-cycloalkylalkoxy, —$NR^7R^8$ or $C_2$-$C_6$-alkoxyalkoxy,

$R^{10}$ is $C_3$-$C_4$-alkinyl, $C_2$-$C_4$-alkenyl, $C_1$-$C_4$-alkyl, $C_2$-$C_4$-alkenyl mono- or polysubstituted by halogen, cyano, $C_1$-$C_4$-alkoxy or —$SO_n$-$C_1$-$C_4$-alkyl, or is $C_1$-$C_4$-alkyl mono- or polysubstituted by halogen, cyano, nitro, $C_1$-$C_4$-alkoxy, $C_1$-$C_4$-haloalkoxy, $C_1$-$C_4$-haloalkylthio, —$SO_n$-$C_1$-$C_4$-alkyl, —T—CX—$R^{18}$,

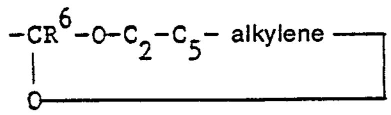

—CO—$R^6$, —CO—$R^9$ or —$SO_2$—$NR^7R^8$, in which n is zero, 1 or 2,

$R^{11}$ is $C_2$-$C_4$-haloalkoxy,

$R^{12}$ is $C_1$-$C_4$-alkyl, $C_1$-$C_4$-haloalkyl or —$NR^{16}R^{17}$,

$R^{13}$ is $C_1$-$C_4$-alkyl, $C_1$-$C_4$-alkoxy, $C_1$-$C_4$-alkylthio, phenyl, di-$C_1$-$C_4$-alkylamino, or a saturated 5- to 7-membered nitrogen heterocycle which is bound by way of the nitrogen atom and which can contain a further hetero atom,

$R^{14}$ and $R^{15}$ independently of one another are $C_1$-$C_4$-alkyl, $C_1$-$C_4$-alkoxy, $C_3$-$C_4$-alkoxyalkoxy, $C_3$-$C_4$-alkenyloxy, $C_1$-$C_6$-alkylthio, $C_1$-$C_4$-alkylamino or di-$C_1$-$C_4$-alkylamino,

$R^{18}$ is $C_1$-$C_4$-alkyl, $C_1$-$C_4$-haloalkyl, $C_1$-$C_4$-alkoxy, $C_1$-$C_4$-alkylthio or —$NR^{16}R^{17}$,

T is oxygen or sulfur,

W is oxygen or =N—O—$R^3$, and

Y is oxygen, sulfur, —SO— or —$SO_2$—;

and the salts of these compounds.

2. Compounds according to claim 1, characterised in that X is —CH=CH—.

3. Compounds according to claim 1, characterised in that Z is oxygen.

4. Compounds according to claim 1, characterised in that $R^1$ is $C_1$-$C_4$-alkoxycarbonyl, nitro, halogen, $C_1$-$C_4$-haloalkyl, $C_1$-$C_4$-haloalkoxy or di-$C_1$-$C_4$-alkylsulfamoyl.

5. Compounds according to claim 1, characterised in that $R^2$ is hydrogen.

6. Compounds according to claim 1, characterised in that $R^3$ is hydrogen.

7. Compounds according to claim 1, characterised in that $R^4$ is halogen, $C_1$-$C_4$-alkoxy or $C_1$-$C_4$-alkyl.

8. Compounds according to claim 1, characterised in that E is the methine bridge.

9. Compounds according to claim 1, characterised in that $R^5$ is $C_1$-$C_4$-alkylcarbonyl, $C_1$-$C_4$-alkoxycarbonyl, $C_1$-$C_4$-alkoxythiocarbonyl, di-$C_1$-$C_4$-alkylcarbamoyl, di-$C_1$-$C_4$-alkylthiocarbamoyl, di-$C_1$-$C_4$-alkoxyphosphonyl, di-$C_1$-$C_4$-alkoxythiophosphonyl, N-pyrrolidinocarbonyl, N-pyrrolidinothiocarbonyl, N-morpholinothiocarbonyl, 2H-1,3,4-triazol-3-yl, 4,5-dihydrothiazol-2-yl, 1-$C_1$-$C_4$-alkyl-imidazol-2-yl, 2-pyridinyl or 2-pyrimidinyl.

10. Compounds according to claim 1, characterised in that X is the ethenylene bridge, Z is oxygen, $R^1$ is $C_1$-$C_4$-alkoxycarbonyl, nitro, halogen, $C_1$-$C_4$-haloalkyl, $C_1$-$C_4$-haloalkoxy or di-$C_1$-$C_4$-alkylsulfamoyl, and $R^2$ and $R^3$ are hydrogen.

11. Compounds according to claim 1, characterised in that $R^4$ is halogen, $C_1$-$C_4$-alkoxy or $C_1$-$C_4$-alkyl, E is the methine bridge, and $R^5$ is $C_1$-$C_4$-alkylcarbonyl, $C_1$-$C_4$-alkoxycarbonyl, $C_1$-$C_4$-alkoxythiocarbonyl, di-$C_1$-$C_4$-alkylcarbamoyl, di-$C_1$-$C_4$-alkylthiocarbamoyl, di-$C_1$-$C_4$-alkoxyphosphonyl, di-$C_1$-$C_4$-alkoxythiophosphonyl, N-pyrrolidinocarbonyl, N-pyrrolidinothiocarbonyl, N-morpholinothiocarbonyl, 1H-1,2,4-triazol-3-yl, 4,5-dihydrothiazol-2-yl, 1-$C_1$-$C_4$-alkyl-imidazol-2-yl, 2-pyridinyl or 2-pyrimidinyl.

12. Compounds according to claim 1, characterised in that X is the ethenylene bridge, Z is oxygen, $R^1$ is $C_1$-$C_4$-alkoxycarbonyl, nitro, halogen, $C_1$-$C_4$-haloalkyl, $C_1$-$C_4$-haloalkoxy or di-$C_1$-$C_4$-alkylsulfamoyl, and $R^2$ and $R^3$ are hydrogen, halogen, $C_1$-$C_4$-alkoxy or $C_1$-$C_4$-alkyl, E is the methine bridge, and $R^5$ is $C_1$-$C_4$-alkylcarbonyl, $C_1$-$C_4$-alkoxycarbonyl, $C_1$-$C_4$-alkoxythiocarbonyl, di-$C_1$-$C_4$-alkylcarbamoyl, di-$C_1$-$C_4$-alkylthiocarbamoyl, di-$C_1$-$C_4$-alkoxyphosphonyl, di-$C_1$-$C_4$-alkoxythiophosphonyl, N-pyrrolidinocarbonyl, N-pyrrolidinothiocarbonyl, N-morpholinothiocarbonyl, 2H-1,2,4-triazol-3-yl, 4,5-dihydrothiazol-2-yl, 1-$C_1$-$C_4$-alkyl-imidazol-2-yl, 2-pyridinyl or 2-pyrimidinyl.

13. N-(2-Methoxycarbonylphenyl-sulfonyl)-N'-(4-methoxy-6-acetylthiomethyl-pyrimidin-2-yl)-urea according to claim 1.

14. N-(2-Methoxycarbonylphenyl-sulfonyl)-N'-(4-methoxy-6-methoxythiocarbonylthiomethyl-pyrimidin-2-yl)-urea according to claim 1.

15. N-(2-Nitrophenyl-sulfonyl)-N'-[4-methoxy-6-(4,5-dihydrothiazol-2-yl-thiomethyl)-pyrimidin-2-yl]-urea according to claim 1.

16. N-(2-Methoxycarbonylphenyl-sulfonyl)-N'-(4-methoxy-6-dimethoxythiophosphonylthiomethyl-pyrimidin-2-yl)-urea according to claim 1.

17. N-(2-Nitrophenyl-sulfonyl)-N'-(4-methoxy-6-diethoxyphosphonylthiomethyl-pyrimidin-2-yl)-urea according to claim 1.

18. N-(2-Methoxycarbonylphenyl-sulfonyl)-N'-(4-methoxy-6-di-n-butyloxythiophosphonyl-thiomethyl-pyrimidin-2-yl)-urea according to claim 1.

19. A process for producing the compounds of the formula I according to claim 1, characterized in that an arylsulfonylisocyanate or arylsulfonylisothiocyanate of the formula II

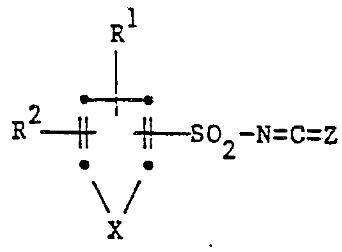

(II)

wherein $R^1$, $R^2$, X and Z have the meanings defined under the formula I, is reacted with an aminopyrimidine or aminotriazine of the formula III

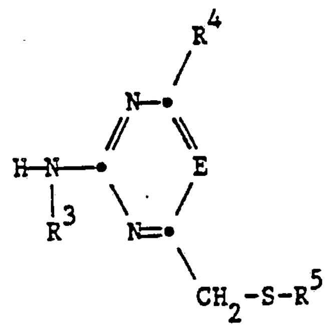

(III)

wherein $R^3$, $R^4$, $R^5$ and E have the meanings defined under the formula I; and the compound obtained is optionally converted into a salt thereof.

20. A process for producing the compounds of the formula I according to claim 1, characterised in that an N-arylsulfonylcarbamate of the formula IV

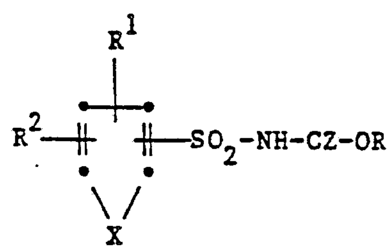

(IV)

wherein $R^1$, $R^2$, X and Z have the meanings defined under the formula I, and R is phenyl, alkyl or substituted phenyl, is reacted with an aminopyrimidine or aminotriazine of the formula III; and the compound obtained is optionally converted into a salt thereof.

21. A process for producing the compounds of the formula I, characterised in that a compound of the formula V

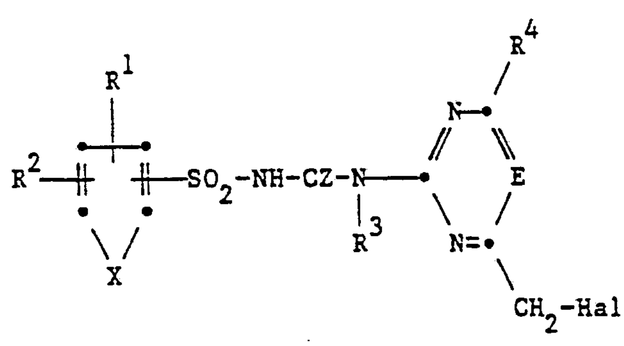

(V)

wherein $R^1$, $R^2$, $R^3$, $R^4$, X, E and Z have the meanings defined under the formula I, and Hal is chlorine or bromine, is reacted with a sulfur salt of the formula VI

$$M^{\oplus}\ S^{\ominus}-R^5 \qquad \text{(VI)}$$

wherein $R^5$ has the meanings defined under the formula I, and $M^{\oplus}$ is an alkali metal cation, alkaline-earth metal cation or ammonium cation ; and the compound obtained is optionally converted into a salt thereof.

22. A process for producing addition salts of the formula I according to any one of claims 19 to 21, characterised in that a sulfonylurea of the formula I is reacted with an amine, with an alkali metal hydroxide or alkaline-earth metal hydroxide or with a quaternary ammonium base.

23. A herbicidal and plant-growth-inhibiting composition, characterised in that it contains, as active ingredient, at least one arylsulfonyl-N'-(4-mercaptomethylpyrimidinyl- or -triazinyl)-urea of the formula I, claim 1, together with carriers and/or other additives.

24. The use of the active substances of the formula I according to claim 1, or of compositions containing them, for controlling undesirable plant growth.

25. The use of the active substances of the formula I according to claim 1, or of compositions containing them, for inhibiting plant growth.

26. The use of the active substances of the formula I, or of compositions containing them, according to claim 25, for the selective pre- or post-emergence controlling of weeds in crops of useful plants.

27. A process for the selective controlling of weeds in crops of useful plants, characterised in that the crops or the cultivated area thereof are treated with an effective amount of an active substance of the formula I, claim 1.

28. Aminopyrimidines and aminotriazines of the formula III

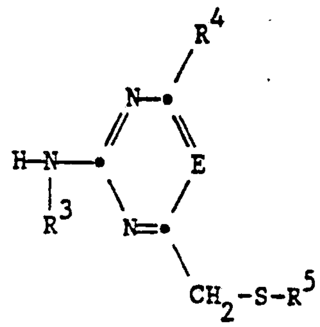

(III)

wherein $R^3$, $R^4$, $R^5$ and E have the meanings defined under the formula I in claim 1.

**Revendications**

1. N-arylsulfonyl-N'-(4-mercaptométhyl-pyrimidinyl- et -triazinyl)-urées de formule I

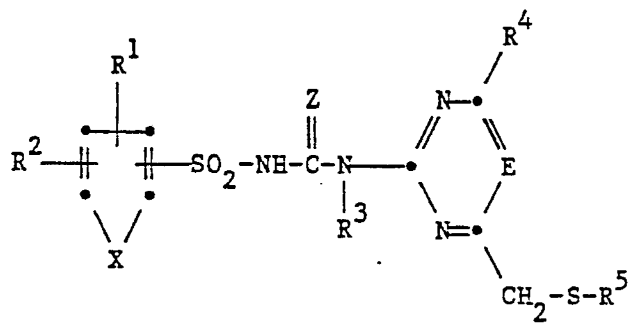

(I)

dans laquelle

E représente l'azote ou —CH=,

X représente l'oxygène, le soufre, un groupe $—NR^3—$, $—N=CR^3—$, —CH = CH— ou

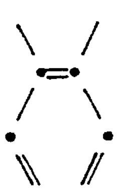

Z représente l'oxygène ou le soufre,

$R^1$ représente l'hydrogène, un halogène, un groupe nitro, éthynyle, —$NR^{16}R^{17}$, —$CR^6$-di-alcoxy en C1-C4,

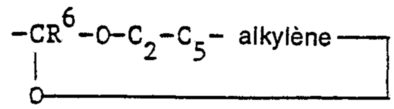

—CW—$R^6$, —$SO_2$—$NR^7R^8$, —CO—$R^9$, —$Y_m$—$R^{10}$, —$SO_2$—$R^{11}$ ou —O—$SO_2$—$R^{12}$, m étant égal à 0 ou 1,

$R^2$ représente l'hydrogène, un halogène, un groupe alkyle en C1-C4, alcoxy en C1-C4, halogénoalkyle en C1-C4 ou nitro,

$R^3$ représente l'hydrogène, un groupe alkyle en C1-C4, alcényle en C3-C4 ou alcoxy en C1-C4,

$R^4$ représente l'hydrogène, un halogène, un groupe alkyle en C1-C4, alcoxy en C1-C4, halogénoalkyle en C1-C4, halogénoalcoxy en C1-C4, alkylthio en C1-C4, alcoxyalkyle en C2-C4, alcoxyalcoxy en C2-C4, cyclopropyle, —$NH_2$, alkylamino en C1-C4, di-(alkyle en C1-C4)-amino ou un hétérocycle azoté saturé à 5-7 chaînons relié par l'intermédiaire de l'atome d'azote et qui peut encore contenir un autre hétéroatome,

$R^5$ représente un groupe cyano, —CZ—$R^{13}$,

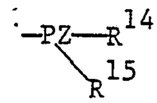

ou un hétérocycle insaturé choisi dans le groupe de l'imidazole, du triazole, de la pyridine, de la pyrimidine, du thiazole, de l'oxazole, du thiadiazole, de l'oxadiazole, de la pyridazine, du thiphène ou du furanne ou de leurs dérivés partiellement hydrogénés, non substitué ou portant un substituant choisi dans le groupe formé par les halogènes, les groupes alkyle en C1-C4, alcoxy en C1-C4 ou halogénoalkyle en C1-C4,

$R^6$ représente l'hydrogène, un groupe alkyle en C1-C4, halogénoalkyle en C1-C4, cycloalkyle en C3-C6, cycloalkylalkyle en C4-C7 ou alcoxyalkyle en C2-C4,

$R^7$ et $R^{16}$ représentent chacun, indépendamment l'un de l'autre, l'hydrogène, un groupe alkyle en C1-C4, cyanalkyle en C1-C4 ou alcoxy en C1-C4,

$R^8$ et $R^{17}$ représentent chacun, indépendamment l'un de l'autre, l'hydrogène, un groupe alkyle en C1-C4, alcényle en C3-C4 ou alcoxy en C1-C4, ou bien

$R^7$ et $R^8$ d'une part, $R^{16}$ et $R^{17}$ d'autre part, indépendamment les uns des autres, forment ensemble et avec l'atome d'azote auquel ils sont reliés un hétérocycle azoté saturé à 5-7 chaînons qui peut encore contenir un autre hétéroatome,

$R^9$ représente un groupe alcoxy en C1-C4, alcényloxy en C3-C6, alcynyloxy en C3-C6, halogénoalcoxy en C2-C6, cyanalcoxy en C1-C4, alkylthio en C1-C4, alcénylthio en C3-C4, alcynylthio en C3-C4, cycloalcoxy en C5-C6, cycloalkylalcoxy en C4-C7, —$NR^7R^8$ ou alcoxyalcoxy en C2-C6,

$R^{10}$ représente un groupe alcynyle en C3-C4, alcényle en C2-C4, alkyle en C1-C4, alkyle en C1-C4 substitué une ou plusieurs fois par des halogènes, des groupes cyano, nitro, alcoxy en C1-C4, halogénoalcoxy en C1-C4, halogénoalkylthio en C1-C4, —$SO_n$—alkyle en C1-C4, —T—CX—$R^{18}$,

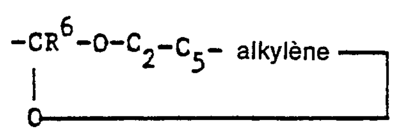

—CO—$R^6$, —CO—$R^9$ ou —$SO_2NR^7R^8$, n étant égal à 0, 1 ou 2,

$R^{11}$ représente un groupe halogénoalcoxy en C2-C4,

$R^{12}$ représente un groupe alkyle en C1-C4, halogénoalkyle en C1-C4 ou —$NR^{16}R^{17}$,

$R^{13}$ représente un groupe alkyle en C1-C4, alcoxy en C1-C4, alkylthio en C1-C4, phényle, di-(alkyle en C1-C4)-amino ou un hétérocycle azoté saturé à 5-7 chaînons relié par l'intermédiaire de l'atome d'azote et qui peut encore contenir un autre hétéroatome,

$R^{14}$ et $R^{15}$ représentent chacun, indépendamment l'un de l'autre, un groupe alkyle en C1-C4, alcoxy en C1-C4, alcoxyalcoxy en C3-C5, alcényloxy en C3-C4, alkylthio en C1-C6, alkylamino en C1-C4 ou di-(alkyle en C1-C4)-amino,

$R^{18}$ représente un groupe alkyle en C1-C4, halogénoalkyle en C1-C4, alcoxy en C1-C4, alkylthio en C1-C4 ou —$NR^{16}R^{17}$,

T représente l'oxygène ou le soufre,

W représente l'oxygène ou le groupe $=N-O-R^3$, et

Y représente l'oxygène, le soufre, un groupe —SO— ou $-SO_2-$,

et les sels de ces composés.

2. Composés selon la rev. 1, caractérisés en ce que X représente le groupe —CH=CH—.

3. Composés selon la rev. 1, caractérisés en ce que Z représente l'oxygène.

4. Composés selon la rev. 1, caractérisés en ce que $R^1$ représente un groupe (alcoxy en C1-C4)-carbonyle, nitro, un halogène, un groupe halogénoalkyle en C1-C4, halogénoalcoxy en C1-C4 ou di-(alkyle en C1-C4)-sulfamoyle.

5. Composés selon la rev. 1, caractérisés en ce que $R^2$ représente l'hydrogène.

6. Composés selon la rev. 1, caractérisés en ce que $R^3$ représente l'hydrogène.

7. Composés selon la rev. 1, caractérisés en ce que $R^4$ représente un halogène, un groupe alcoxy en C1-C4 ou alkyle en C1-C4.

8. Composés selon la rev. 1, caractérisés en ce que E représente le pont méthine.

9. Composés selon la rev. 1, caractérisés en ce que $R^5$ représente un groupe (alkyle en C1-C4)-carbonyle, (alcoxy en C1-C4)-carbonyle, (alcoxy en C1-C4)-thiocarbonyle, di-(alkyle en C1-C4)-carbamoyle, di-(alkyle en C1-C4)-thiocarbamoyle, di-(alcoxy en C1-C4)-phosphonyle, di-(alcoxy en C1-C4)-thiophosphonyle, N-pyrrolidinocarbonyle, N-pyrrolidinothiocarbonyle, N-morpholinothiocarbonyle, 2H-1,3,4-triazole-3-yle, 4,5-dihydrothiazole-2-yle, 1-(alkyle en C1-C4)-imidazole-2-yle, 2-pyridinyle ou 2-pyrimidinyle.

10. Composés selon la rev. 1, caractérisés en ce que X représente le pont éthénylène, Z représente l'oxygène, $R^1$ représente un groupe (alcoxy en C1-C4)-carbonyle, nitro, un halogène, un groupe halogénoalkyle en C1-C4, halogénoalcoxy en C1-C4 ou di-(alkyle en C1-C4)-sulfamoyle et $R^2$ et $R^3$ représentent l'hydrogène.

11. Composés selon la rev. 1, caractérisés en ce que $R^4$ représente un halogène, un groupe alcoxy en C1-C4 ou alkyle en C1-C4, E représente le pont méthine et $R^5$ un groupe (alkyle en C1-C4)-carbonyle, (alcoxy en C1-C4)-carbonyle, (alcoxy en C1-C4)-thiocarbonyle, di-(alkyle en C1-C4)-carbamoyle, di-(alkyle en C1-C4)-thiocarbamoyle, di-(alcoxy en C1-C4)-phosphonyle, di-(alcoxy en C1-C4)-thiophosphonyle, N-pyrrolidinocarbonyle, N-pyrrolidinothiocarbonyle, N-morpholinothiocarbonyle, 1H-1,2,4-triazole-3-yle, 4,5-dihydrothiazole-2-yle, 1-(alkyle en C1-C4)-imidazole-2-yle, 2-pyridinyle ou 2-pyrimidinyle.

12. Composés selon la rev. 1, caractérisés en ce que X représente le pont éthénylène, Z l'oxygène, $R^1$ un groupe (alcoxy en C1-C4)-carbonyle, nitro, un halogène, un groupe halogénoalkyle en C1-C4, halogénoalcoxy en C1-C4 ou di-(alkyle en C1-C4)-sulfamoyle et $R^2$ et $R^3$ représentent l'hydrogène, des halogènes, des groupes alcoxy en C1-C4 ou alkyle en C1-C4, E représente le pont méthine et $R^5$ représente un groupe (alkyle en C1-C4)-carbonyle, (alcoxy en C1-C4)-carbonyle, (alcoxy en C1-C4)-thiocarbonyle, di-(alkyle en C1-C4)-carbamoyle, di-(alkyle en C1-C4)-thiocarbamoyle, di-(alcoxy en C1-C4)-phosphonyle, di-(alcoxy en C1-C4)-thiophosphonyle, N-pyrrolidinocarbonyle, N-pyrrolidinothiocarbonyle, N-morpholinothiocarbonyle, 2H-1,2,4-triazole-3-yle, 4,5-dihydrothiazole-2-yle, 1-(alkyle en C1-C4)-imidazole-2-yle, 2-pyridinyle ou 2-pyrimidinyle.

13. La N-(2-méthoxycarbonylphényl-sulfonyl)-N'-(4-méthoxy-6-acétylthiométhylpyrimidine-2-yl)-urée selon la rev. 1.

14. La N-(2-méthoxycarbonylphényl-sulfonyl)-N'-(4-méthoxy-6-méthoxythiocarbonylthiométhyl-pyrimidine-2-yl)-urée selon la rev. 1.

15. La N-(2-nitrophényl-sulfonyl)-N'-[4-méthoxy-6-(4,5-dihydrothiazole-2-yl-thiométhyl)-pyrimidine-2-yl]-urée selon la rev. 1.

16. La N-(2-méthoxycarbonylphényl-sulfonyl)-N'-(4-méthoxy-6-diméthoxythiophosphonylthiométhyl-pyrimidine-2-yl)-urée selon la rev. 1.

17. La N-(2-nitrophényl-sulfonyl)-N'-(4-méthoxy-6-diéthoxyphosphonylthiométhyl-pyrimidine-2-yl)-urée selon la rev. 1.

18. La N-(2-méthoxycarbonylphényl-sulfonyl)-N'-(4-méthoxy-6-di-n-butyloxythiophosphonyl-thiométhyl-pyrimidine-2-yl)-urée selon la rev. 1.

19. Procédé de préparation des composés de formule I selon la rev. 1, caractérisé en ce que l'on fait réagir un arylsulfonylisocyanate ou -isothiocyanate de formule II

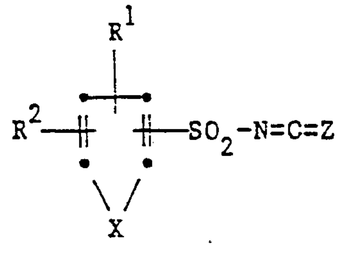

(II)

dans laquelle $R^1$, $R^2$, X et Z ont les significations indiquées en référence à la formule I, avec une aminopyrimidine ou -triazine de formule III

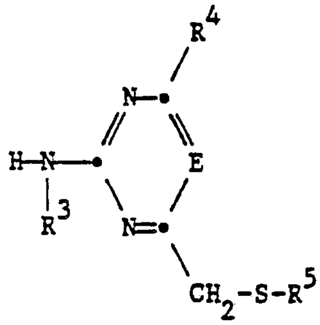

(III)

dans laquelle $R^3$, $R^4$, $R^5$ et E ont les significations indiquées en référence à la formule I, et le cas échéant, on convertit en les sels.

20. Procédé de préparation des composés de formule I selon la rev. 1, caractérisé en ce que l'on fait réagir un N-arylsulfonylcarbamate de formule IV

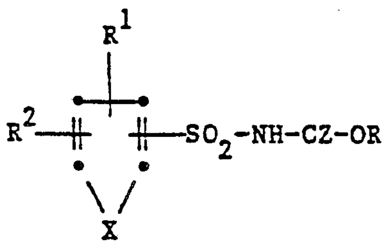

(IV)

dans laquelle $R^1$, $R^2$, X et Z ont les significations indiquées en référence à la formule I et R représente un groupe phényle, alkyle ou phényle substitué, avec une aminopyrimidine ou -triazine de formule III, et le cas échéant, on convertit en les sels.

21. Procédé de préparation des composés de formule I caractérisé en ce que l'on fait réagir un composé de formule V

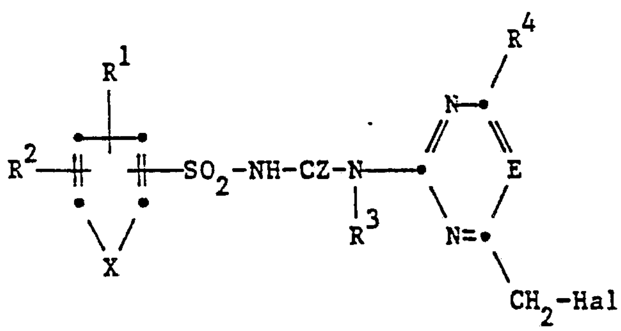

(V)

dans laquelle $R^1$, $R^2$, $R^3$, $R^4$, X, E et Z ont les significations indiquées en référence à la formule I et Hal représente le chlore ou le brome, avec un sulfure de formule VI

$$M^{\oplus}S^{\ominus}—R^5 \qquad (VI),$$

dans laquelle $R^5$ a les significations indiquées en référence à la formule I et M⊕ représente un cation alcalin, alcalino-terreux ou d'ammonium, et le cas échéant, on convertit en les sels.

22. Procédé de préparation des sels de formule I formés par addition selon l'une des rev. 19 à 21, caractérisé en ce que l'on fait réagir une sulfonylurée de formule I avec une amine, un hydroxyde de métal alcalin ou alcalino-terreux ou une base d'ammonium quaternaire.

23. Un produit herbicide et inhibiteur de la croissance des végétaux, caractérisé en ce qu'il contient en tant que substance active, avec des véhicules et/ou d'autres additifs, au moins une arylsulfonyl-N'-(4-mercaptométhylpyrimidinyl- ou -triazinyl)-urée de formule I, rev. 1.

24. L'utilisation des substances actives de formule I selon la rev. 1 ou de produits en contenant dans la lutte contre les croissances de végétaux indésirables.

25. L'utilisation des substances actives de formule I selon la rev. 1 ou de produits en contenant pour l'inhibition de la croissance de végétaux.

26. L'utilisation des substances actives de formule I ou de produits en contenant selon la rev. 25 pour la lutte sélective en pré-levée ou post-levée contre les mauvaises herbes dans les cultures de végétaux utiles.

27. Procédé pour combattre sélectivement les mauvaises herbes dans les cultures de végétaux utiles, caractérisé en ce que l'on traite les cultures ou les aires de culture par une quantité efficace d'une substance active de formule I, rev. 1.

28. Aminopyrimidines et -triazines de formule III

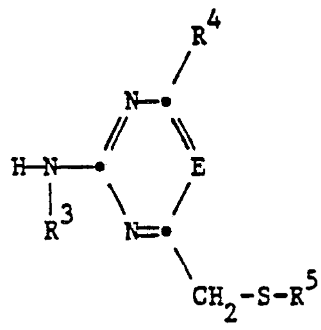

(III)

dans laquelle $R^3$, $R^4$, $R^5$ et E ont les significations indiquées en référence à la formule I dans la rev. 1.